# EUROPEAN PATENT APPLICATION

(11) **EP 4 299 736 A2**
(43) Date of publication of application: **03.01.2024**
(21) Application number: 23168045.5
(22) Date of filing: 14.04.2023
(51) Int. Cl.: C12N 9/78, A61K 38/50

(54) **HYDROLASES AND USES THEREOF**

(30) Priority: 15.04.2022 LT 2022514
(71) Applicant: Vilnius University, 01513 Vilnius (LT)
(72) Inventor: Meskys, Rolandas, Vilnius (LT); Urbeliene, Nina, Vilnius (LT); Tauraite, Daiva, Vilnius (LT); Tiskus, Matas, Vilnius (LT); Preitakaite, Viktorija, Vilnius (LT)
(74) Representative: Petniunaite, Jurga

(57) **Abstract**

The present invention is provided hydrolases originating from prokaryotic, archaea and eukaryotic cells, capable of regioselectively converting *N*⁴-acylcytidine, *N*⁴-alkylcytidine, *N*⁴-carboxycytidine, *S*⁴-alkylthiouridine and *O*⁴-alkoxyuridine derivatives to uridine derivatives. The present disclosure provides using these polypeptides in the processes: 1) for making chemical uridine derivatives from *N*⁴-acylcytidines, *N*⁴-alkylcytidines, *N*⁴-carboxycytidine, *S*⁴-alkylthiouridines and *O*⁴-alkoxyuridine compounds; 2) for making pyrimidine compounds such as intermediates in the production active pharmaceutical drugs; 3) for the prodrug activation; 4) in combination with *N*⁴-acylcytidines, *N*⁴-alkylcytidines, *S*⁴-alkylthiouridines and *O*⁴-alkoxyuridines for the selection of enzymes.

## Description

### FIELD OF THE INVENTION

This invention relates to the field of biochemistry, more specifically to enzymes called hydrolases and their use in modifications of *N*⁴-acylcytidines, *N*⁴-alkylcytidines, *N*⁴-carboxycytidines, *S*⁴-alkylthiouridines and *O*⁴-alkoxyuridine compounds.

### BACKGROUND OF THE INVENTION

The removal of an amino group from a molecule is called deamination, and enzymes that catalyze this reaction are called deaminases. By definition deaminases belong to the family of hydrolases, those acting on carbon-nitrogen bonds other than peptide bonds, specifically in cyclic amidines. After the treatment of deaminases ammonia is released. In living organisms, one of the most well-known deamination processes is the deamination of the nucleobases - cytosine, adenine and guanine. The deamination of pyrimidine, in particular cytidine/2'-deoxycytidine, cytosine or mono-/di-/tri-phosphorylated cytidine compounds, or nucleobases incorporated in DNA/RNA, are catalyzed by the different specific pyrimidine deaminases. In addition, deamination of purine bases adenine and guanine is performed by the specific purine deaminases. Two classes of cytidine deaminases (CDA) have been found in nature: a homodimeric (D-CDA) class, for example, *Escherichia coli, Arabidopsis thaliana, Klebsiella pneumoniae* cytidine deaminases and a homotetrameric class (T-CDA), for example, Homo *sapiens* and *Bacillus subtilis* CDAs. Both classes of CDAs contain one catalytic zinc ion per subunit. Cytidine deaminase [EC 3.5.4.5] is an enzyme of the pyrimidine salvage pathway catalyzing the hydrolytic deamination of cytidine and 2'-deoxycytidine to uridine and 2'-deoxyuridine, respectively. Additionally, it is known that homodimeric and homotetrameric cytidine deaminases catalyze deamination reactions of different *N*⁴-unsubstituted cytidine derivatives. Furthermore, it is known, that *E. coli* cytidine deaminase (encoded by *cdd* gene) catalyzes the hydrolysis of *N*⁴-methylcytidine to uridine (Cohen and Wolfenden, 1971) and cytokinin deaminase catalyzes the conversion of *N*⁶-substituted adenine derivatives to adenine (Goble et al., 2011). As a result, an appropriate amine is released in the both reactions.

At present it is known, that deamination hydrolysis reactions of cytosine, adenine or guanine into uracil, hypoxanthine and xanthine, respectively, releasing ammonia in the process, could be spontaneous. But spontaneous deamination/deamidation reactions of acyl/alkyl nucleosides are not known. Moreover, deamination or deamidation of the larger *N*⁴-acylated/*N*⁴-alkylated group of cytidine derivatives catalyzed by hydrolases is not demonstrated up to now. Additionally, it is not demonstrated that enzymes belonging to the deaminase class hydrolyze *S*⁴-alkylthiouridine and *O*⁴-alkyluridine derivatives. The chemical deamination of pyrimidine nucleosides is known, but it is demonstrated that the chemical deamination reaction requires harsh conditions - high temperature and pressure. The methods of chemical deamination are described in a few publications only (Clark et al., 2005; Nowak and Robins, 2005).

Fluorinated analogues of 4-acylated or 4-alkylated cytidine derivatives have potential use in chemotherapy. The fluoropyrimidines exert its antitumor effects through several mechanisms including inhibition of RNA synthesis and function, inhibition of thymidylate synthase activity, and incorporation into DNA, leading to DNA strand breaks. Unfortunately, most anticancer drugs have a low therapeutic index - a small difference between doses that cause anticancer effects and doses that cause toxicity. This is determined by several cellular enzymes. One of them is CDA - enzyme played an important role in the sensitivity/resistance of cancer cells to treatment with cytidine analogs (Jansen at al. 2011). CDA is involved in the metabolism of nucleoside analogues such as gemcitabine (Bjånes et al., 2020), capecitabine (Walko and Lindley, 2005), cytarabine (Liu et al., 2016) and azacytidine (Murakami et al., 2019), which are widely used as anticancer drugs. By catalyzing the deamination of nucleoside analogues, CDA induces their deactivation (gemcitabine, cytarabine, azacytidine) or their activation (capecitabine). It is shown that pre-anticancer drug capecitabine initially passes intact through the gastrointestinal mucosa and is converted to 5'-deoxy-5-fluorocytidine ribonucleoside (5'DFCR) by carboxylesterase and then to 5'-deoxy-5-fluorouridine (5'-DFUR) by cytidine deaminase. Finally, thymidine phosphorylase converts 5'-DFUR to the active drug. This occurs in both tumor and normal tissue and causes adverse effects (Shelton et al., 2016) (Alvarez et al., 2012). To date, it has not been estimated that an intestinal microbiota could contain cytidine deaminases that may convert capecitabine to a toxic fluorouridine in the intestine, resulting in additionally induced side effects.

Various methods have been developed to reduce the toxicity of chemotherapeutic antimetabolites. One of them is a method called enzyme-prodrug system that could help improve the efficacy and safety of conventional cancer chemotherapies. In this approach, cancer cells are first transfected with a gene that can express an enzyme with ability to convert a nontoxic prodrug into its active cytotoxic form. As a result, the activated prodrug could kill the transfected cancer cells (Malekshah et.al. 2016). At present quite a lot of prodrug-enzymes systems have already been proposed, examples of patented enzymes/prodrug systems are: (1) yeast or bacterial cytosine deaminase converting the prodrug 5-fluorocytosine into the cytotoxic chemotherapeutic agent 5-fluorouracil (5- FU) (US010035983B2) (2) an isocytosine deaminase/5-fluoroisocytosine enzyme/prodrug pair for gene-directed cancer therapy (LT6626B); (3) genetically engineered modified human deoxycytidine kinase (dCK) with enhanced activity towards nucleoside analogues used in cancer chemotherapy (WO2009143048A2, US8349318 B2); (3) polypeptides with nitroreductase activity use in combination with prodrugs (WO2000047725 A1, WO2005049845 A2, WO2006103452, WO2015075475 A1, WO9512678 A2); (4) nicotinamide riboside kinase (US20080206221) and (5) cytochrome P 450 or cytochrome P 450 reductase (WO9945126 A2).

Notwithstanding offerings of therapy systems, new strategies are still needed due to cancer cell resistance to drugs and drug toxicity to organisms. A proposed enzyme-prodrug system has some advantages: 1) the 4^{th} position of heterocyclic bases of fluoropyrimidines can be very miscellaneous (-N-R, -S-R or -O-R residues, when R could be alkyl/acyl or carbamate residues). This feature expands the spectrum of possible drugs, moreover, this provides a possibility to combine different prodrug compounds together with one enzyme and, hence, to avoid a development of resistance to the drug(s); 2) the prodrug can consist of two anticancer compounds that are liberated after treatment by the hydrolase, hence, a double anticancer effect could be expected; 3) the large spectrum of prodrug compounds increases the number of potential targets in cancer cells resulting in decrease of the possibility of development of drug resistance. Moreover, the described enzymes combined with already known fluoropyrimidine prodrugs may increase their anticancer effects; 4) proposed enzymes hydrolyze 4-(N/S/O)-substituted fluoropyrimidines directly to 4-unsubstituted fluorouridine derivatives without participation of amidohydrolases or carboxylesterases of human body. It expands the spectrum of protective groups of prodrugs, because it does not require additional enzymes.

Thymidine kinase from herpesvirus is a sub-family of thymidine kinases. Its presence in herpesvirus-infected cells is used to activate a range of antivirals against herpes infection, and thus specifically target the therapy towards infected cells only. Some drugs are specifically directed against dividing cells. They can be used against tumours and viral diseases (both against retrovirus and against other virus), as the diseased cells replicate much more frequently than normal cells and also against some non-malignant diseases related to excessively rapid cell replication (for instance psoriasis). The VZV thymidine kinase, in contrast to the mammalian enzyme, selectively monophosphorylates specific pyrimidine compounds. Moreover, pyrimidine analogues are converted to cytotoxic or cytostatic metabolites in specific mammalian cells which are genetically modified to selectively express VZV thymidine kinase (EP 0 415 731 B1). To decrease side effects, hydrolases of the present invention can be used for conversion of non-toxic prodrugs to generate the substrates of VZV thymidine kinase.

The closest analogue to the invention is described in Nowak and Robins, 2005. The cytidine and 2'-deoxycytidine derivatives are hydrolytically deamidated in superheated water/DME (dimethoxyethane) solutions in an oil bath at 125 °C or in a methanol-ammonia mixture. In contrast, the hydrolases described in the present invention can be used for the chemoenzymatic deamination/deamidation of cytidine derivatives *in vitro* in an environmentally friendly conditions (aqueous inorganic salt solutions pH 7-8) without the use of aggressive organic solvents, elevated pressure and high temperatures (>100 °C). Furthermore, such discovered unexpected reactions can change attitudes towards many living processes and has practical applications. For example, fluorinated acyl-nucleosides or alkyl-nucleosides, referred to as anti-metabolites, together with specific hydrolases, could form an enzyme-prodrug system for chemotherapy or could be use as antiviral system.

### SUMMARY OF THE INVENTION

The present invention relates to process during which polypeptides convert the substrate compounds - *N*⁴-substituded cytidine, *S*⁴-substituted thiouridine, *O*⁴-substituted derivatives or *N*⁴-substituded pseudoisocytidine of general formulas **1, 4, 6, 8, 9** (*Scheme 1*, *2*, *3*, *4*, *5*) to its corresponding product compounds of general formulas **2, 3, 5, 7, 10.**

At first aspect, the invention relates to an isolated hydrolase selected from the group consisting of SEQ ID NO: 1-41.

In one aspect, the hydrolase polypeptides described herein have amino acid sequences with one or more amino acid differences compared to each other.

In other aspect, the hydrolase polypeptides described herein have amino acid sequences with one or more amino acid differences as compared to a wild-type hydrolase or as compared to an engineered hydrolase. The one or more amino acid differences result in at least one improved property of the enzyme for a defined substrate. Generally, the hydrolase polypeptides described herein are engineered hydrolase polypeptides having one or more improved properties as compared to the naturally-occurring wild-type hydrolase enzymes obtained from SEQ ID NO: 1. Improvements in an enzyme property of the engineered hydrolase polypeptides include increases in enzyme activity and enlarged substrate range.

In other aspect, a hydrolase polypeptide of the present invention is at least 70% identical to the reference sequence of SEQ ID NO: 1, and has, at the position corresponding to the indicated position of SEQ ID NO: 1 at least one of the following amino acid differences: the amino acid at position 51 is a nonpolar aliphatic amino acid selected from among glycine, alanine, leucine and valine or the amino acid at position 51 is an polar amino acid selected from among threonine and serine; the amino acid at position 81 is a nonpolar aliphatic amino acid selected from among glycine, and alanine, the amino acid at position 83 is a polar charged or neutral amino acid selected from among arginine, histidine, lysine, asparagine, glutamine, aspartic acid, glutamic acid, serine and threonine, the amino acid at position 83 is a nonpolar aliphatic amino acid selected from alanine and glycine; the amino acid at position 84 amino acid is a nonpolar aliphatic amino acid selected from alanine, leucine, valine, isoleucine, glycine, methionine and proline, or the amino acid at position 84 amino acid is a polar amino acid selected from asparagine, glutamine, aspartic acid, glutamic acid, serine, and threonine; the amino acid at position 85 is a nonpolar aliphatic amino acid selected from among glycine, alanine, leucine, isoleucine, valine and methionine or the amino acid at position 85 is a polar amino acid selected from among serine or threonine; the amino acid at position 127 is either an polar amino acid selected from among threonine, serine or the charged amino acid histidine, or amino acid at position 127 is a nonpolar amino acid alanine, leucine, valine, isoleucine, glycine, methionine or cysteine; the amino acid at position 128 is either a nonpolar aliphatic amino acid selected from among alanine, leucine, valine, isoleucine, glycine and methionine or the amino acid at position 128 is a polar amino acid selected from among serine, threonine or lysine and arginine; the amino acid at position 129 is a nonpolar aromatic amino acid selected from among tyrosine, tryptophan and phenylalanine, or amino acid at position 129 is the polar amino acid selected from among serine, threonine, histidine, glutamic or aspartic acid; the amino acid at position 130 is a nonpolar aliphatic or aromatic amino acid selected from among glycine, alanine, leucine, valine, isoleucine, glycine, methionine and tyrosine, tryptophan, and phenylalanine; the amino acid at position 126 is a nonpolar aliphatic or aromatic amino acid selected from among alanine, leucine, valine, isoleucine, glycine, methionine, phenylalanine, tyrosine and tryptophan.

The level of sequence identity between a query and a subject sequence is preferably determined using a sequence alignment program, such as the ClustalW (Thompson, et. al. 1994).

In certain embodiments, the invention provides hydrolase polypeptides capable of converting *N*⁴-acylcytidine compounds of general formula (**1**) to the corresponding products - uridine derivative of formula (**2**) and amide of general formula (**3**), as depicted in **Scheme 1** below: 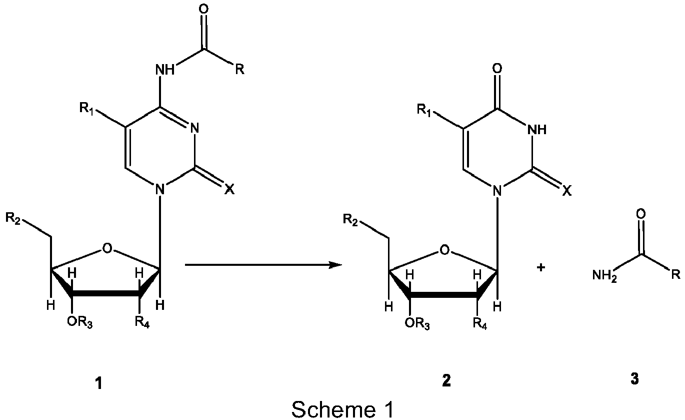
Wherein R is C1-C10 alkyl, C1-C10 substituted alkyl, -(CH₂)n-Ph, or substituted -(CH₂)n-Ph; wherein in said -(CH₂)n-Ph, n = 0, 1, 2, 3 to 10, Ph is phenyl; a carbon chain of said substituted alkyl is independently substituted by one or two or three hydroxyl group or carboxyl group; in said substituted -(CH₂)n-Ph, n = 0, 1, 2, 3 to 10, and a carbon chain or a phenyl ring of which is independently substituted by one or two or hydroxyl group or carboxyl group;
Wherein X is S, O;
Wherein R₁ is C1-C10 alkyl, C1-C10 substituted alkyl or F;
Wherein R₂ is -OH, -PO₄²⁻, C1-C10 alkyl, C1-C10 substituted alkyl;
Wherein R₃: H;
Wherein R₄: H, OH, C1-C10 alkyl, C1-C10 substituted alkyl or F.

In certain embodiments, the disclosure provides hydrolase polypeptides capable of converting N(O,S)4-alkyl-pyrimidine compounds of general formula (**4**) to the corresponding products uridine derivative of general formula (**2**) and compound of general formula (**5**), as depicted in **Scheme 2** below: 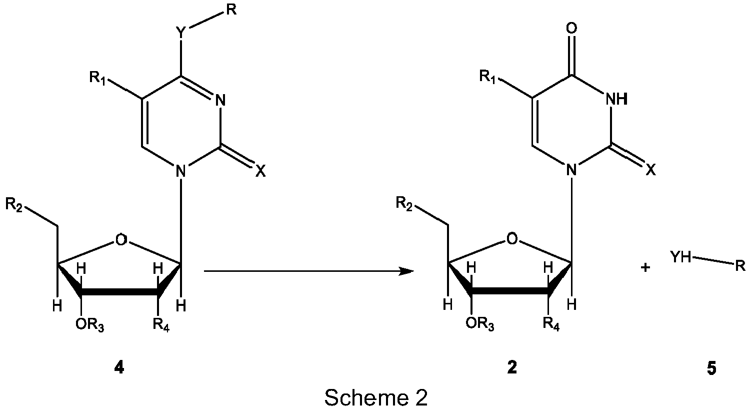
Wherein Y is NH, S or O;
Wherein R is C1-C10 alkyl, C1-C10 substituted alkyl, -(CH₂)n-Ph, or substituted -(CH₂)n-Ph; wherein in said -(CH₂)n-Ph, n = 0, 1, 2, 3 to 10, Ph is phenyl; a carbon chain of said substituted alkyl is independently substituted by one or two or three hydroxyl group or carboxyl group; in said substituted -(CH2)n-Ph, n = 0, 1, 2, 3 to 10, and a carbon chain or a phenyl ring of which is independently substituted by one or two or hydroxyl group or carboxyl group;
Wherein X is S, O;
Wherein R₁ is C1-C10 alkyl, C1-C10 substituted alkyl or F;
Wherein R₂ is -OH, -PO₄²⁻, C1-C10 alkyl, C1-C10 substituted alkyl;
Wherein R₃: H;
Wherein R₄: H, OH, C1-C10 alkyl, C1-C10 substituted alkyl or F.

In certain embodiments, the disclosure provides hydrolase polypeptides capable of converting *N*⁴-acyl-pseudoisocytidine compounds of Formula (**6**) to the corresponding products - uridine derivative of general formula (**7**) and amide of general formula (**3**), as depicted in **Scheme 3** below: 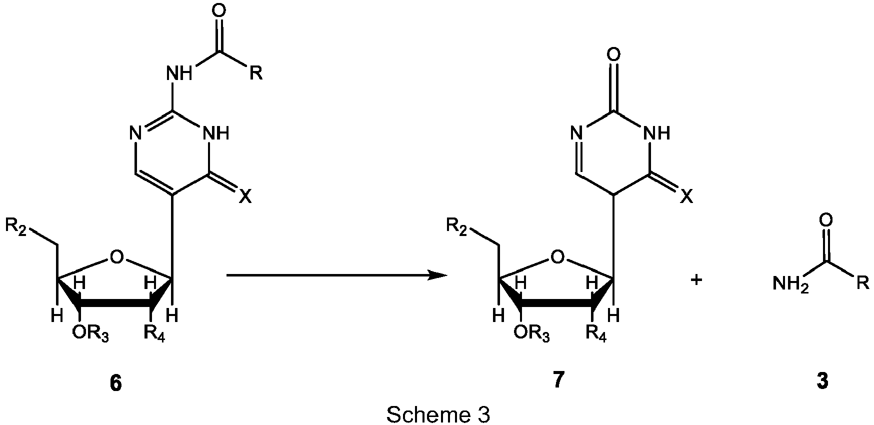
Wherein R is C1-C10 alkyl, C1-C10 substituted alkyl, -(CH₂)n-Ph, or substituted -(CH₂)n-Ph; wherein in said -(CH₂)n-Ph, n = 0, 1, 2, 3 to 10, Ph is phenyl;
a carbon chain of said substituted alkyl is independently substituted by one or two or three hydroxyl group or carboxyl group; in said substituted -(CH₂)n-Ph, n = 0, 1, 2, 3 to 10, and a carbon chain or a phenyl ring of which is independently substituted by one or two or hydroxyl group or carboxyl group;
Wherein X is S, O;
Wherein R₂: H, OH;
Wherein R₃: H;
Wherein R₄: H, OH, C1-C10 alkyl, C1-C10 substituted alkyl or F

In certain embodiments, the disclosure provides hydrolase polypeptides capable of converting N(O,S)2-alkyl-pseudoisocytidine compounds of general formula (**8**) to the corresponding products - pseudoisouridine derivative of general formula (**7**) and compound of general formula (**5**), as depicted in **Scheme 4** below: 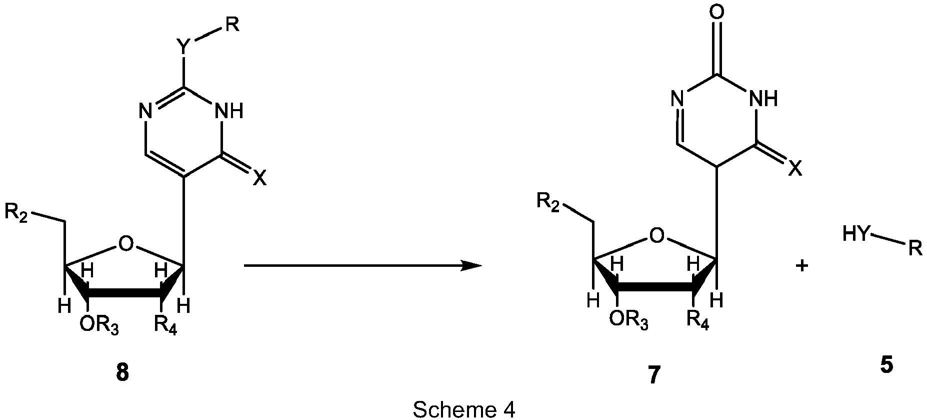
Wherein Y is NH, S or O;
Wherein R is C1-C10 alkyl, C1-C10 substituted alkyl, -(CH₂)n-Ph, or substituted -(CH₂)n-Ph; wherein in said -(CH₂)n-Ph, n = 0, 1, 2, 3 to 10, Ph is phenyl; a carbon chain of said substituted alkyl is independently substituted by one or two or three hydroxyl group or carboxyl group; in said substituted -(CH₂)n-Ph, n = 0, 1, 2, 3 to 10, and a carbon chain or a phenyl ring of which is independently substituted by one or two or hydroxyl group or carboxyl group;
Wherein X is S, O;
Wherein R₂: H, OH;
Wherein R₃: H;
Wherein R₄: H, OH, C1-C10 alkyl, C1-C10 substituted alkyl or F.

In certain embodiments, the disclosure provides hydrolase polypeptides capable of converting *N*⁴-Alkoxycarbonylcytidine compound of general formula (**9**) to the corresponding products - uridine derivative of general formula (**2**) and carbamate of general formula (**10**), as depicted in **Scheme 5** below: 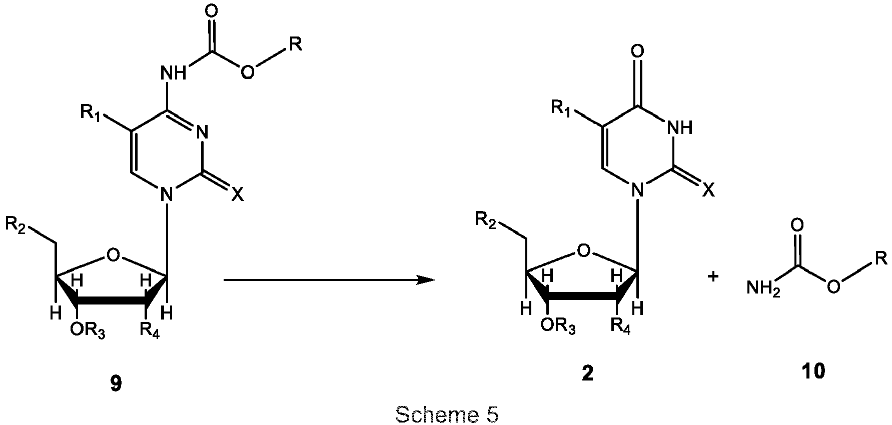
Wherein R is C1-C10 alkyl, C1-C10 substituted alkyl, -(CH2)n-Ph, or substituted -(CH2)n-Ph; wherein in said -(CH₂)n-Ph, n = 0, 1, 2, 3 to 10, Ph is phenyl; a carbon chain of said substituted alkyl is independently substituted by one or two or three hydroxyl group or carboxyl group; in said substituted -(CH₂)n-Ph, n = 0, 1, 2, 3 to 10, and a carbon chain or a phenyl ring of which is independently substituted by one or two or hydroxyl group or carboxyl group;
Wherein X is S, O;
Wherein R₁ is C1-C10 alkyl, C1-C10 substituted alkyl or F;
Wherein R₂ is -OH, -PO₄²⁻, C1-C10 alkyl, C1-C10 substituted alkyl;
Wherein R₃: H;
Wherein R₄: H, OH, C1-C10 alkyl, C1-C10 substituted alkyl or F.

In some embodiments, the method converting the substrate compound (**1**, **4, 6, 8, 9**) to its corresponding product compound (**2**, **3, 5, 7, 10**), comprises contacting or incubating the substrate with at least one of the hydrolase polypeptides SEQ ID NO: 1 - 41 disclosed herein under reaction conditions suitable for converting the substrate to the product.

In some embodiments of the above methods, the substrate is converted to the product in greater than about 95%, greater than about 97%, or greater than about 99% yield, wherein the hydrolase polypeptide comprises a sequence that corresponds to SEQ ID NO: 1-41.

In some embodiments of the above methods, at least about 95% of the substrate is converted to the product in less than 24 hours when carried out with greater than about 100 g/L of substrate and less than about 5 g/L of the polypeptide, wherein the polypeptide comprises an amino acid sequence corresponding to SEQ ID NO: 1 - 41.

In one embodiment, the invention provides a method of at least inhibiting, and typically killing replicating or non-replicating, transfected or transduced mammalian cells and bystander cells through the following steps: (a) transfecting or transducing targeted mammalian cells with a nucleic acid encoding SEQ ID NO: 1 - 41 or providing such enzyme directly in proximity to the targeted cells; and (b) contacting the targeted cells expressing or provided with the SEQ ID NO: 1 - 41 cleavage enzyme with a substrate for the enzyme to produce a toxic pirimidine base there by killing the targeted cells and also bystander cells not expressing or containing the cleavage enzyme. Thus, in the presence of substrate, the cleavage enzyme produces a toxic product.

In another embodiment, the invention provides a functional selection or screening of enzymes, when the target enzymes catalyze the reactions of conversion of *N*⁴-acyl/*N*⁴-alkyl/*N*⁴-alkoxycarbonylcytidine derivatives, *S*⁴-alkyluridine or *O*⁴-alkoxyuridine derivatives, which are not substrates of hydrolases of SEQ ID NO: 1 - 41, into *N*⁴-acyl/*N*⁴-alkyl/*N*⁴-alkoxycarbonylcytidine derivatives, *S*⁴-alkyluridine or *O*⁴-alkoxyuridine derivatives, which can be hydrolyzed by one of hydrolases of SEQ ID NO: 1 - 41 into the reaction products, which are uridine derivatives according the **Scheme 6** below: 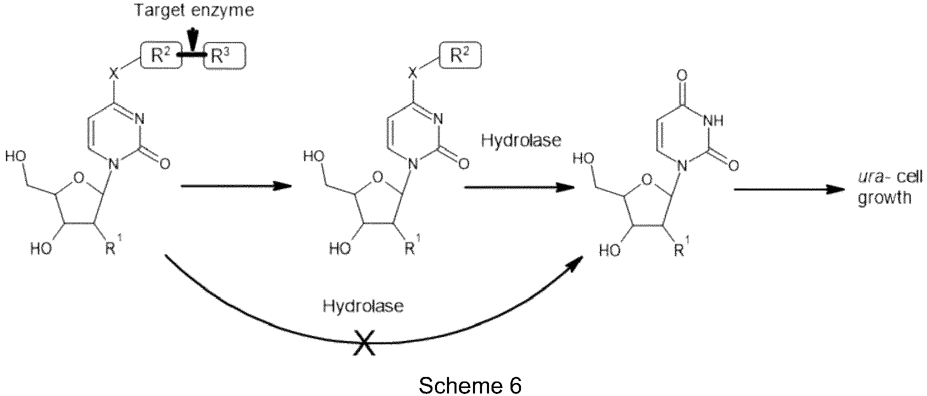
Wherein R₁ is H, OH, NH₂;
R₂ is a fragment, which can be removed by one of hydrolases of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41;
R₃ is a fragment, which is removed by the target enzyme;
Target enzyme is the enzyme, which is being screened or selected;
Hydrolase is one of hydrolases of SEQ ID NO: 1 - 41;
Wherein X is NH, S, O.

The uridine derivatives can be further detected by chemical or physical methods or can be used as a compound supporting the growth of the uridine auxothrophic cells.

In another embodiment, the invention provides a method when hydrolases are used for the genome editing in CRISPR-Cas9 system. The principal of process is that: DNA with a target cytidine or her analogue (modificated cytidine) at a locus specified by a guide RNA is bound by Cas9 nuclease, which mediates local DNA strand separation. Cytidine deamination by a tethered converts the single-stranded target C→U. The resulting G:U heteroduplex can be permanently converted to an A:T bp following DNA replication or DNA repair.

### BRIEF DESCRIPTION OF FIGURES

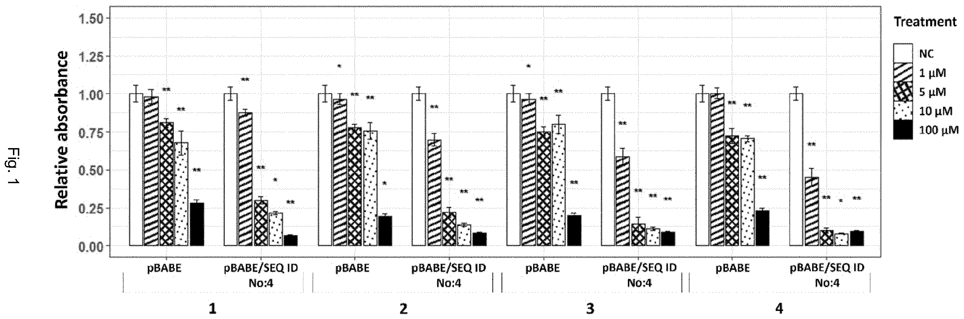

**Figure 1****.** Cytotoxic activity of enzyme-prodrug system. HCT116 cells transduced with Seq ID NO:4-encoding vector pBABE/Seq ID No:4, or control vector pBABE-Puro (designated as pBABE) were treated with 1 µM, 5 µM, 10 µM and 100 µM of different prodrugs for the 24 h. Statistical significance indicated by *p*-values, where the symbol * designates *p* < 0.05, whereas the symbol ** designates *p* < 0.01 with respect to untreated cells (negative control (NC)). HCT116 cells were treated with prodrugs: **1** - 5-fluoro- *N*⁴-acetylcytidine, **2** - 5-fluoro-*N*⁴-benzoylcytidine, **3** - 5-fluoro- *N*⁴-pivaloylcytidine, **4** - 5-fluoro-4-((1*H*-indol-3-yl)propionyl)-cytidine.

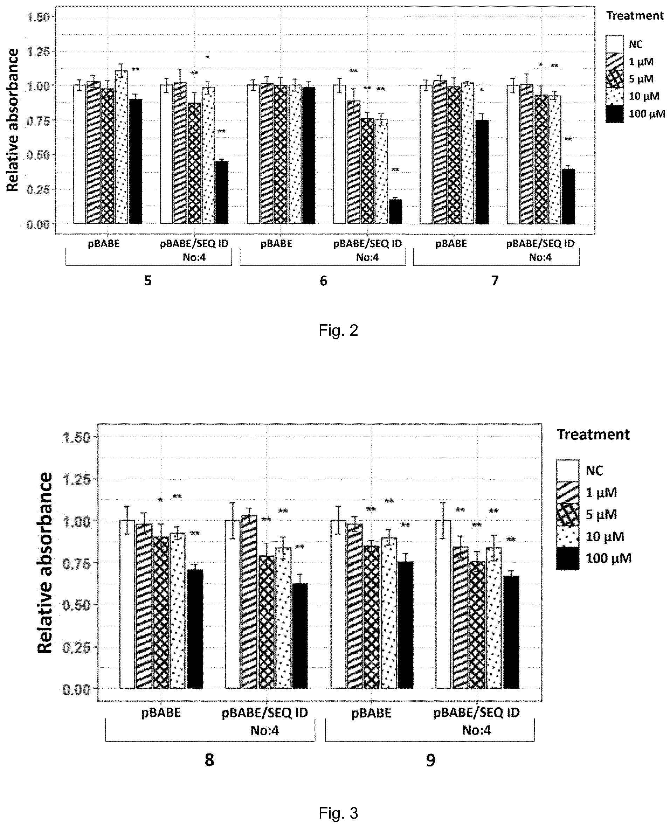

**Figure 2****.** Cytotoxic activity of enzyme-prodrug systemHCT116 cells transduced with Seq ID NO: 4-encoding vector pBABE/Seq ID NO: 4, or control vector pBABE-Puro (designated as pBABE) were treated with 1 µM, 5 µM, 10 µM and 100 µM of different prodrugs for the 24 h. Statistical significance indicated by *p*-values, where the symbol * designates *p* < 0.05, whereas the symbol ** designates *p* < 0.01 with respect to untreated cells (negative control (NC)). HCT116 cells were treated with prodrugs: **5** - 5-fluoro-*N*⁴-benzyloxyuridine, **6** - 5-fluoro-*N*⁴-butoxyuridine, **7** - 5-fluoro-*N*⁴-methoxyuridine.
**Figure 3****.** Cytotoxic activity of enzyme-prodrug system. HCT116 cells transduced with Seq ID NO: 4-encoding vector pBABE/Seq ID NO: 4, or control vector pBABE-Puro (designated as pBABE) were treated with 1 µM, 5 µM, 10 µM and 100 µM of different prodrugs for the 24 h. Statistical significance indicated by *p*-values, where the symbol * designates *p* < 0.05, whereas the symbol ** designates *p* < 0.01 with respect to untreated cells (negative control (NC)). HCT116 cells were treated with prodrugs: **8** -5-fluoro-*N*⁴-metyllthiouridine, **9** - 5-fluoro-*N*⁴-etyilthiouridine.

### DETAILED DESCRIPTION OF THE INVENTION

The term hydrolase and/or cytidine deaminase as used therein, unless otherwise specified, refers to a polypeptide that catalyzes the irreversible hydrolytic deamination of *N*⁴-unsubstituted cytidine derivatives (cytidine and pseudoisocytidine and modified nucleosides' analogues), *N*⁴-unsubstituted deoxycytidine derivatives (2'deoxycytidine, pseudoiso-2'-deoxycytidine 2',3'-dideoxycytidine, 2',5'-dideoxycytidine and modified nucleosides' analogues), *N*⁴-acyl cytidine derivatives, *N*⁴-acyl-2'-deoxycytidine derivatives, *N*⁴-alkyl cytidine derivatives, *N*⁴-alkyl-2'-deoxycytidine derivatives, *N*⁴-alkoxycarbonylcytidine derivatives, *S*⁴-alkyl-thiouridine derivatives or *S*⁴-alkyl-thio-2'-deoxyuridine derivatives, *O*⁴-alkyluridine derivatives or *O*⁴-alkoxy-2'-deoxyuridine to uridine and 2'-deoxyuridine compounds, respectively and includes at least one of declared herein specified gene sequence fragments SEQ ID NO: 1-41.

As used herein, the term "nucleoside" means a ribonucleoside or deoxyribonucleoside, or a modified form thereof, or an analogue thereof. The terms "derivatives " or "compound" and other word having the same root and used in combination with the term "cytidine" or "uridine" are mean that the nucleosides have additional chemical groups that is not present in natural nucleosides cytidine or uridine. Any hydroxyl group of carbohydrate of the nucleoside (e.g., ribose, deoxyribose, arabinose or other monosaccharide) may be replaced with methyl, ethyl, azido groups or other functional groups of organic compounds. The 5' ends of carbohydrate may be monophosphorylated. Nucleoside can also be composed of ribose and deoxyribose as an alternative monosaccharides including 2'-O-methyl-, 2'-*O*-allyl-, 2'-*O*-ethyl-, 2'-*O*-propyl-, 2'-methoxyethyl-, 2'-fluoro-, 2'-amino-, 2'-azido-pentoses, α-anomers, other aldopentoses (e.g., arabinose, xylose, lyxose), pyranoses, acyclic analogues. Nucleosides also can be L-(+) stereoisomers.

The term "*N*⁴-unsubstituted cytidine" or "*N*⁴ unsubstituted -2'-deoxycytidine" derivatives means that the nucleosides can have additional chemical groups in all positions, except *N*⁴ atom.

The term "*N*⁴- substituted cytidine" and "*N*⁴- substituted 2'-deoxycytidine" derivatives means that the nucleosides have acyl, alkyl or carbamate groups onto *N*⁴ position, also derivatives can have additional chemical groups in all positions. The term "*S*⁴-substituted thio-uridine" derivatives means that the nucleosides have alkyl groups onto *S*⁴ position, also derivatives can have additional chemical groups in all positions. The term "*O*⁴-substituted uridine" derivatives means that the nucleosides have alkyl groups onto *O*⁴ position, also derivatives can have additional chemical groups in all positions.

The term "alkyl", as used herein, unless otherwise specified, refers to a *N*⁴-alkyl, 4-thioalkyl (S4-alkyl) or 4-alkoxy (O4-alkyl) straight or branched or aromatic hydrocarbon. In one embodiment, the alkyl group is a primary, secondary, or tertiary hydrocarbon. In one embodiment, the alkyl group includes two to ten carbon atoms, i.e., C2 to C10 alkyl. In one embodiment, the alkyl group is aromatic or heterocyclic.

The term "acyl", as used herein, unless otherwise specified, refers to an *N*⁴ amide group straight or branched or aromatic hydrocarbon. In one embodiment, the acyl group is a primary, secondary, or tertiary amide. In one embodiment, the acyl group includes two to ten carbon atoms, i.e., C2 to C10 alkyl. In one embodiment, the acyl group is aromatic or heterocyclic.

As used herein the term "inhibiting" is an alteration of a normal physiological activity. Specifically, inhibiting is defined as lysing, reducing proliferation, reducing growth, increasing or decreasing the expression or rate of degradation of a gene, RNA, protein, lipid, or other metabolite, inducing apoptosis or other cell death mechanisms, or increasing, decreasing, or otherwise altering the function of a protein or nucleic acid.

"Screening of enzymes" refers to evaluation of every protein for the desired property, while "selection of enzymes" automatically eliminates nonfunctional variants. Selection or screening of enzymes can be performed from enzyme libraries *in vivo* or *in vitro.* The term "enzyme library" refers to a set of proteins or genes inserted into expression vectors or cells producing the enzymes. The enzyme library can be a metagenomic library or a mutant library. The metagenomic library can be constructed from various sources of environmental sample, such as soil, water, sludge, and etc. Specifically, the selected or screened enzyme may be of any enzyme group, for example, such as oxidoreductases, hydrolases, lyases, etc.

### EXAMPLES OF IMPLEMENTATION OF THE INVENTION

### Example 1. Synthesis of alkylated thiouridine and alkoxyuidine derivatives

The thiouridine and alkoxyuridine compounds using in this invention are synthesized according to Synthetic Scheme:

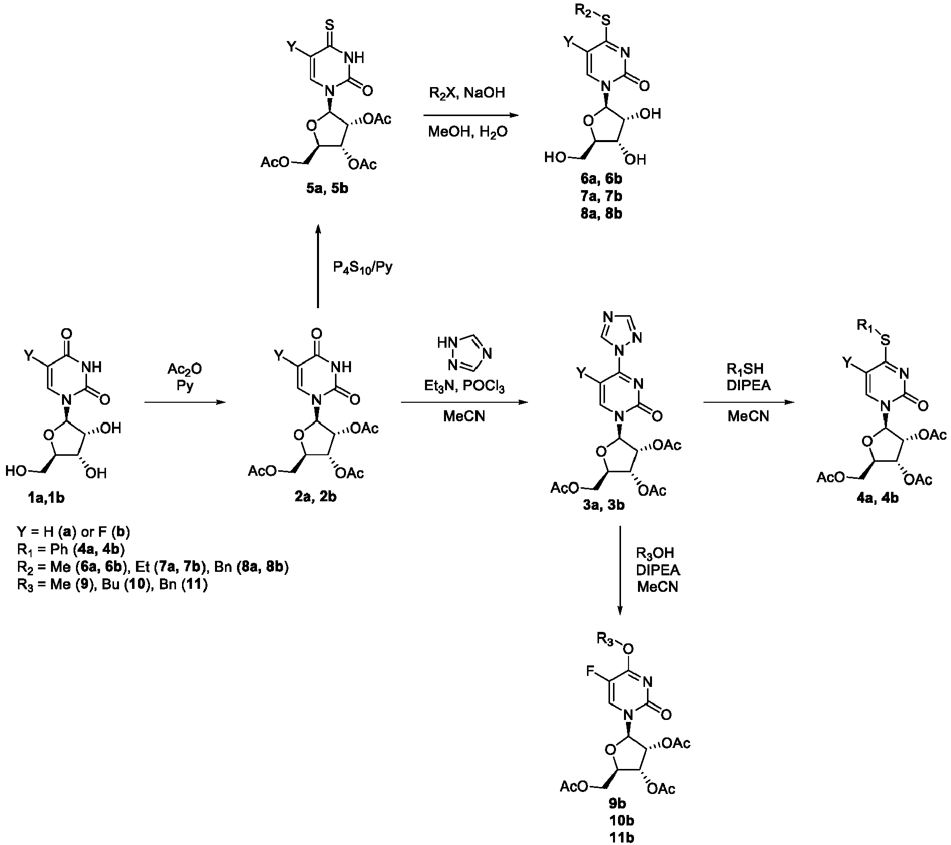

### Acetylation of uridine (1a) and 5-fluorouridine (1b). General procedure:

Acetic anhydride (6 eq.) was added dropwise to a 0.5 M solution of a substrate (1 eq.) in pyridine at room temperature. After 16 h, the reaction was quenched by the addition of methanol and the mixture was concentrated under reduced pressure by co-evaporating it with toluene at 40-45 °C (4 to 5 times). Traces of pyridine were removed by extraction: the crude mixture was dissolved in dichloromethane (1 g/25 mL) and it was washed twice with 1M HCl and once with water, then brine. The organic phase was dried over Na₂SO₄, filtered, and concentrated under reduced pressure.

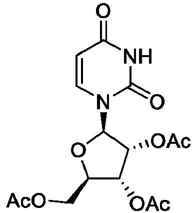

### 2', 3', 5'-Tri-O-acetyluridine (2a)

The acetylation of uridine (1a) was done on 41 mmol scale (10 g). The product was isolated as colourless glassy oil (15.1 g, 99 %) and was used in further reactions without additional purification. (Gong, Y. et. al, 2020)

(2a) R*_{f}* = 0.6 [DCM:MeOH (14:1); ¹H NMR (400 MHz, CDCl₃) δ 9.44 (s, 1H), 7.39 (d, *J* = 8.2 Hz, 1H), 6.04 (d, *J* = 4.6 Hz, 1H), 5.79 (dd, *J* = 8.2, 2.1 Hz, 1H), 5.37 - 5.27 (m, 2H), 4.40 - 4.27 (m, 3H), 2.13 (s, 3H), 2.12 (s, 3H), 2.09 (s, 3H); ¹³C NMR (100 MHz, CDCl₃) δ 170.3, 169.8 (2C), 163.0, 150.4, 139.4, 103.6, 87.6, 80.1, 72.8, 70.3, 63.3, 20.9, 20.6, 20.5.

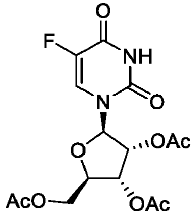

### 2',3',5'-Tri-O-acetyl-5-fluorouridine (2b)

The acetylation of 5-fluorouridine was done on 5.8 mmol (1.51 g) scale. The product **2b** was isolated as colourless glassy oil (2.2 g, 98 %) and was used in further reactions without additional purification.

(**2b**): R*_{f}* = 0.7 [DCM:MeOH (10:1)]; ¹H NMR (400 MHz, CDCl₃) δ 9.46 (d, *J* = 4.8 Hz, 1H), 7.61 (d, *J* = 6.0 Hz, 1H), 6.11 - 6.06 (m, 1H), 5.36 - 5.27 (m, 2H), 4.43 - 4.28 (m, 3H), 2.10 (d, *J* = 0.8 Hz, 3H), 2.17 (s, 3H), 2.13 (s, 3H); ¹³C NMR (100 MHz, CDCl₃) δ 170.2, 169.8 (2C), 156.6 (d, *J* = 27.0 Hz), 149.1, 141.0 (d, *J* = 239.4 Hz), 123.3 (d, *J* = 34.6 Hz), 87.2, 80.3, 72.9, 70.2, 63.1, 20.9, 20.6, 20.5.

### 2', 3', 5'-Tri-O-acetyl-4-(1,2,4-triazol-1-yl)uridine (3a) and 2',3',5'-Tri-O-acetyl-5-fluoro-4-(1,2,4-triazol-1-yl)uridine (3b)

Compounds **3a** and **3b** were synthesized using literature procedure (Milecki, J. et. al., 2011)

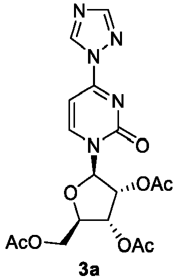

### 2', 3', 5'-Tri-O-acetyl-4-(1,2,4-triazol-1-yl)uridine (3a)

The compound **3a** was isolated as a colourless oil (1.1 g, 85 % yield from 1.14 g (3.08 mmol) of 2', 3', 5'-tri-O-acetyluridine (**2a**)) (Gao, Y. et Al., 2003).

(**3a**) R*_{f}* = 0.2 [DCM:MeOH:acetone (50:1:1)]; ¹H NMR (400 MHz, DMSO-*d₆*) δ 9.45 (s, 1H), 8.48 (d, *J* = 7.3 Hz, 1H), 8.43 (s, 1H), 7.08 (d, *J* = 7.2 Hz, 1H), 6.02 (d, *J* = 3.6 Hz, 1H), 5.56 (dd, *J* = 6.0, 3.6 Hz, 1H), 5.38 (t, *J* = 6.3 Hz, 1H), 4.44 - 4.34 (m, 2H), 4.29 (dd, *J* = 12.3, 5.6 Hz, 1H), 2.09 (s, 3H), 2.07 (s, 6H); ¹³C NMR (100 MHz, DMSO-*d₆*) δ 170.0, 169.3, 169.2, 159.2, 154.2, 153.4, 149.4, 143.9, 94.7, 90.7, 79.5, 72.9, 69.4, 62.7, 20.6, 20.3 (2C).

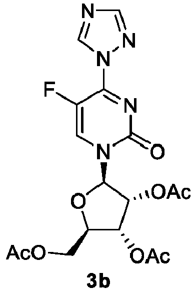

### 2', 3', 5'-Tri-O-acetyl-5-fluoro-4-(1,2,4-triazol-1-yl)uridine (3b)

The compound **3b** was unstable during column chromatography purification. Therefore, it was prepared and, after isolating it from the liquid-liquid extraction, immediately used as a crude in further reactions (**4b**, **9b**, **10b**, **11b**).

(**3b**): R*_{f}* = 0.4-0.5 [DCM:MeOH (30:1)]; ¹H NMR (400 MHz, CDCl₃) δ 9.25 (s, 1H), 8.41 (d, *J* = 6.6 Hz, 1H), 8.23 (s, 1H), 6.13 (d, *J* = 3.6 Hz, 1H), 5.46 (dd, *J* = 5.2, 3.5 Hz, 1H), 5.30 (d, *J* = 6.2 Hz, 1H), 4.51 (dt, *J* = 6.2, 2.8 Hz, 1H), 4.48 - 4.40 (m, 2H), 2.19 (s, 3H), 2.15 (s, 4H), 2.10 (s, 3H); ¹⁹F NMR (375 MHz, CDCl₃) δ -156.77 (d, *J* = 6.6 Hz).

### General procedure for 4-phenylthiouridine 4a, 5-fluoro-4-phenylthiouridine 4b and 4-alkoxy-5-fluorouridines 9b, 10b, 11b:

DIPEA (Hünigs base, 5 eq, 0.5 mmol, 87 µL) and thiophenol (5 eq, 0.5 mmol, 51 µL) or R₃OH (5 eq, 0.5 mmol) were added to a 0.1 M solution of 4-(1,2,4-triazol-1-yl)uridine **3a** or **3b** (1 eq, 0.1 mmol) in acetonitrile at room temperature. After 16 h, the reaction mixture was diluted with water (5 mL) and extracted with dichloromethane (2×10 mL). Combined organic phases were washed with brine (1×5 mL), then dried over Na₂SO₄, filtered, and concentrated under reduced pressure. Products were purified by column chromatography (DCM: MeOH 200:1 to 30:1).

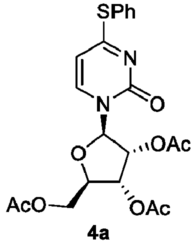

### 2', 3', 5'-Tri-O-acetyl-4-S-phenylthiouridine (4a)

The compound **4a** was isolated as a colorless oil (115 mg, 89 % yield from 0.282 mmol of 4-(1,2,4-triazol-1-yl)uridine **3a**).

(**4a**): R*_{f}* = 0.3 [DCM:MeOH (50:1)]; ¹H NMR (400 MHz, CDCl₃) δ 7.62 - 7.53 (m, 3H), 7.52 - 7.39 (m, 3H), 6.05 (d, *J* = 4.3 Hz, 1H), 5.92 (d, *J* = 7.2 Hz, 1H), 5.34 (dd, *J* = 5.6, 4.3 Hz, 1H), 5.31 - 5.23 (m, 1H), 4.41 - 4.33 (m, 1H), 4.36 - 4.26 (m, 2H), 2.09 (s, 3H), 2.07 (s, 3H), 2.07 (s, 3H); ¹³C NMR (100 MHz, CDCl₃) δ 181.3, 170.2, 169.7, 169.6, 152.9, 140.5, 135.8, 130.5, 130.0, 127.3, 102.1, 89.3, 80.0, 73.8, 69.9, 63.0, 20.9, 20.6 (2C; HRMS (ESI) calculated for C₂₁H₂₃O₈N₂S (M+H)⁺: 463.1170; found 463.1170.

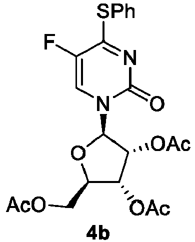

### 2', 3', 5'-Tri-O-acetyl-5-fluoro-4-S-phenylthiouridine (4b)

The product 4b was isolated as a colorless glassy oil (110 mg, 61 % yield over 2 steps from 0.376 mmol of 2',3',5'-tri-O-acetyl-5-fluorouridine (2b)).

(4b): R*_{f}* = 0.4 [DCM:MeOH (30:1)]; ¹H NMR (400 MHz, DMSO-*d₆*) δ 8.32 (d, *J* = 5.6 Hz, 1H), 7.62 - 7.48 (m, 5H), 5.82 (d, *J* = 3.7 Hz, 1H), 5.46 (dd, *J* = 6.2, 3.8 Hz, 1H), 5.32 (t, *J* = 6.1 Hz, 1H), 4.38 - 4.22 (m, 3H), 2.06 (s, 3H), 2.04 (s, 3H), 2.04 (s, 3H); ¹³C NMR (100 MHz, DMSO-*d₆*) δ 170.0, 169.2, 169.1, 168.2 (d, *J=* 17.1 Hz), 150.7, 142.2 (d, *J* = 239.1 Hz), 135.7 (2C), 130.4, 129.6 (2C), 128.3 (d, *J* = 34.2 Hz), 124.1, 90.1, 79.4, 72.6, 69.2, 62.7, 20.5, 20.2 (2C); ¹⁹F NMR (375 MHz, DMSO-*d₆*) δ -157.82, -157.84; HRMS (ESI) calculated for C₂₁H₂₂O₈N₂SF (M+H)⁺: 481.1075; found 481.1073.

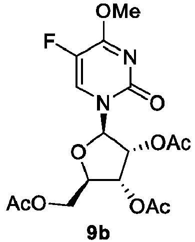

### 2', 3', 5'-Tri-O-acetyl-5-fluoro-4-methoxyuridine (9b)

The product 9b was isolated as a colorless glassy oil (640 mg, 69 % yield over 2 steps from 2.3 mmol of 2',3',5'-tri-O-acetyl-5-fluorouridine (**2b**)).

(**9b**): R*_{f}* = 0.4 [DCM:MeOH (30:1)]; ¹H NMR (400 MHz, CDCl₃) δ 7.81 (d, *J* = 5.7 Hz, 1H), 6.15 (dd, *J* = 4.4, 1.5 Hz, 1H), 5.36 - 5.32 (m, 1H), 5.32 - 5.25 (m, 1H), 4.44 - 4.31 (m, 3H), 4.07 (s, 3H), 2.16 (s, 3H), 2.11 (s, 3H), 2.09 (s, 3H); ¹³C NMR (100 MHz, CDCl₃) δ 170.1, 169.7, 169.6, 163.1 (d, *J* = 13.0 Hz), 153.4, 137.2 (d, *J* = 248.9 Hz), 126.4 (d, *J* = 32.0 Hz), 88.3, 80.0, 73.7, 69.7, 62.7, 55.5, 20.9, 20.6 (2C); ¹⁹F NMR (375 MHz, CDCl₃) δ -167.60 (d, *J* = 6.0 Hz). LCMS; HRMS (ESI) calculated for C₁₆H₁₉O₉N₂FNa (M+Na)⁺: 425.0967; found 425.0964.

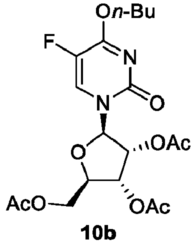

### 2', 3', 5'-Tri-O-acetyl-5-fluoro-4-butoxyuridine (10b)

The product **10b** was isolated as a colorless oil (53 mg, 32 % yield over 2 steps from 0.37 mmol of 2',3',5'-tri-O-acetyl-5-fluorouridine (**2b**)).

(**10b**): R*_{f}* = 0.25 [DCM:MeOH (30:1)]; ¹H NMR (400 MHz, CDCl₃) δ 7.79 (d, *J* = 5.8 Hz, 1H), 6.14 (dd, *J* = 4.4, 1.5 Hz, 1H), 5.34 (t, *J* = 4.9 Hz, 1H), 5.28 (t, *J* = 5.3 Hz, 1H), 4.52 - 4.40 (m, 2H), 4.43 - 4.21 (m, 3H), 2.16 (s, 3H), 2.10 (s, 3H), 2.09 (s, 3H), 1.77 (p, *J* = 6.9 Hz, 2H), 1.44 (h, *J* = 7.4 Hz, 2H), 0.95 (t, *J* = 7.4 Hz, 3H); ¹³C NMR (100 MHz, CDCl₃) δ 170.1, 169.7, 169.6, 163.0 (d, *J* = 13.0 Hz), 153.5, 137.2 (d, *J* = 248.9 Hz), 126.3 (d, *J* = 32.2 Hz), 88.3, 79.9, 73.7, 69.7, 68.6, 62.7, 30.5, 20.8, 20.6 (2C), 19.1, 13.8; LCMS; HRMS (ESI) calculated for C₁₉H₂₅O₉N₂FNa (M+Na)⁺: 467.1436; found 467.1431.

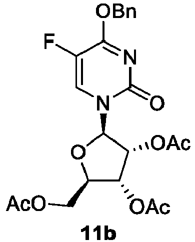

### 2', 3', 5'-Tri-O-acetyl-5-fluoro-4-benzyloxyuridine (11b)

The product **11b** was isolated as a colorless oil (40 mg, 23 % yield over 2 steps from 0.37 mmol of 2',3',5'-tri-O-acetyl-5-fluorouridine (**2b**)).

(**11b**): R*_{f}* = 0.25-0.35 [DCM:MeOH (30:1)]; ¹H NMR (400 MHz, CDCl₃) δ 7.84 (d, *J* = 5.7 Hz, 1H), 7.50 - 7.43 (m, 2H), 7.43 - 7.33 (m, 3H), 6.15 (dd, *J* = 4.4, 1.5 Hz, 1H), 5.50 (s, 2H), 5.36 (dd, *J* = 5.4, 4.3 Hz, 1H), 5.29 (t, *J* = 5.4 Hz, 1H), 4.44 - 4.31 (m, 3H), 2.16 (s, 3H), 2.12 (s, 3H), 2.09 (s, 3H); ¹³C NMR (100 MHz, CDCl₃) δ 170.1, 169.7, 169.6, 162.6 (d, *J* = 13.1 Hz), 153.3, 137.1 (d, *J* = 249.2 Hz), 134.7, 128.9, 128.8 (4C), 126.7 (d, *J* = 32.0 Hz), 88.4, 79.9, 73.8, 70.0, 69.7, 62.7, 20.8, 20.61 (2C); HRMS (ESI) calcd for C₂₂H₂₃O₉N₂FNa (M+Na)⁺: 501.1280; found 501.1278.

Deacetylation of derivatives 2', 3', 5'-Tri-O-acetyl-5-fluoro-4-methoxyuridine (**9b**), 2', 3', 5'-Tri-O-acetyl-5-fluoro-4-butoxyuridine (**10b**) and 2', 3', 5'-Tri-O-acetyl-5-fluoro-4-benzyloxyuridine (**11b**) were performed in the reaction mixture containing 40 mM potassium phosphate buffer, pH 7.5, 0.5 mg/ml esterase SVG1 (Urbeliene, N. et. al, 2019) and 12-20 mg/ml derivative (from 100 mM Stock in DMSO). Reaction mixture were incubated at 37°C up to 3 h.

**Synthesis of thiones 5a and 5b** can be accomplished directly from the uridines **1a** and **1b,** as described below, excluding the extraction procedure for the acetylation step. Otherwise, it can be followed from the (*) mark.

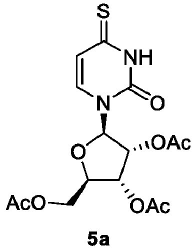

### 2',3',5'-Tri-O-acetyl-4-thiouridine (5a)

Acetic anhydride (172 mmol, 16.3 mL) was added in a dropwise manner to a solution of uridine (**1a**, 41 mmol, 10 g) in pyridine (120 mL) and left to stir overnight at room temperature. The reaction was quenched with methanol (20 mL) and evaporated under reduced pressure. Most of the pyridine was removed by co-evaporation with toluene (5×30 mL). 2', 3', 5'-Tri-O-acetyluridine (**2a**) was collected as a colorless oil (16.9 g) and (*) without further purification it was dissolved in dioxane (250 mL). It was followed by addition of phosphorus pentasulfide (91.2 mmol, 20.3 g) and subsequent reflux. After 2 h, the reaction mixture was cooled down and filtered. The remaining solids were washed with DCM (3×50 mL) and the filtrate was concentrated under reduced pressure. The remaining crude mixture was redissolved in DCM (200 mL), filtered through a pad of Celite (washing it with additional portion of DCM, 100 mL) and once again concentrated under reduced pressure. The resulting oil was subjected to column chromatography (two columns: 1. DCM: MeOH 100:1 to 25:1 and 2. THF:hex 1:2.3 to 1:1.8). 2',3',5'-Tri-O-acetyl-4-thiouridine (**5a**) was isolated as a colorless glassy oil (10 g, 63 % yield).

(**5a**) (Kaleta, Z. et. Al.; 2006): R*_{f}* = 0.65 [DCM:MeOH (19:1)]; ¹H NMR (400 MHz, CDCl₃) δ 10.12 (s, 1H), 7.23 (d, *J* = 7.7 Hz, 1H), 6.42 (dd, *J* = 7.7, 1.7 Hz, 1H), 5.97 (d, *J* = 4.9 Hz, 1H), 5.39 - 5.28 (m, 2H), 4.41 - 4.29 (m, 3H), 2.13 (s, 3H), 2.11 (s, 3H), 2.10 (s, 3H); ¹³C NMR (100 MHz, CDCl₃) δ 189.9, 170.2, 169.7 (2C), 147.6, 133.7, 114.1, 88.1, 80.2, 73.0, 70.2, 63.1, 20.9, 20.6, 20.5.

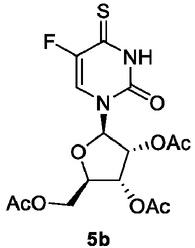

### 2',3',5'-tri-O-acetyl-5-fluoro-4-thiouridine (5b)

2',3',5'-tri-O-acetyl-5-fluoro-4-thiouridine (**5b**) was synthesized following the procedure used for 2',3',5'-tri-O-acetyl-4-thiouridine (**5a**). Reaction was done using 5-fluorouridine (**1b**, 6.37 mmol, 1.67 g). The desired product **5b** was isolated as a yellow oil (2.5 g, 97%) which was unstable in a solution as well as when kept neat.

(**5b**) (Wenska, G.; 2002): R*_{f}* = 0.5 [DCM:MeOH (19:1)]; ¹H NMR (400 MHz, CDCl₃) δ 10.48 (s, 1H), 7.55 (d, *J* = 4.3 Hz, 1H), 6.02 (dd, *J* = 4.7, 1.4 Hz, 1H), 5.36 (t, *J* = 5.2 Hz, 1H), 5.34 - 5.24 (m, 1H), 4.43 - 4.30 (m, 2H), 2.16 (s, 3H), 2.11 (s, 6H); ¹³C NMR (100 MHz, CDCl₃) δ 180.3 (d, *J* = 31.2 Hz), 170.2, 169.8, 169.7, 147.5 (d, *J* = 227.3 Hz), 146.8, 119.2 (d, *J* = 41.3 Hz), 87.9, 80.3, 73.1, 69.9, 62.9, 20.8, 20.6, 20.5; ¹⁹F NMR (375 MHz, CDCl₃) δ -144.57 (d, *J* = 4.1 Hz).

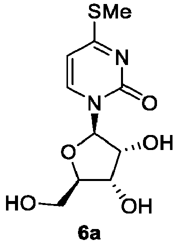

### 4-S-Methylthiouridine (6a)

lodomethane (5.5 mmol, 324 µL) was added to a solution of 2',3',5'-tri-O-acetyl-4-thiouridine (**5a**, 3.1 mmol, 1.18 g) in water-methanol (1:5, 30 mL). It was followed by slow addition of 1M NaOH (3 mL, aq.) over 20 min. After 20 min of stirring at room temperature, reaction was quenched by adding acetic acid (3 mmol, 172 µL). Reaction mixture was concentrated under reduced pressure and subjected to column chromatography (DCM:MeOH 14:1 to 11:1). 4-S-Methylthiouridine (**6a**) was isolated as a white amorphous solid (370 mg, 43 %).

(**6a**): R*_{f}* = 0.3 [DCM:MeOH (9:1)]; ¹H NMR (400 MHz, MeOD-*d₄*) δ 8.31 (d, *J* = 7.1 Hz, 1H), 6.47 (d, *J* = 7.2 Hz, 1H), 5.85 (d, *J* = 2.0 Hz, 1H), 4.21 - 4.11 (m, 2H), 4.12 - 4.03 (m, 1H), 3.94 (dd, *J* = 12.4, 2.4 Hz, 1H), 3.78 (dd, *J* = 12.4, 2.9 Hz, 1H), 2.53 (s, 3H); ¹³C NMR (100 MHz, MeOD-*d₄*) δ 180.5, 156.4, 142.0, 104.9, 92.9, 85.9, 76.5, 70.1, 61.4, 12.9; HRMS (ESI) calcd for C₁₀H₁₅O₅N₂S (M+H)⁺: 275.0696; found 275.0691.

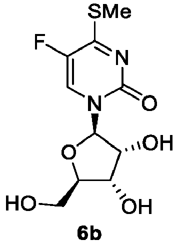

### 5-Fluoro-4-S-methylthiouridine (6b)

5-Fluoro-4-S-methylthiouridine (**6b**) was synthesized following the procedure for 4-S-methylthiouridine (**6a**). The reaction was done using 2',3',5'-tri-O-acetyl-5-fluoro-4-thiouridine (**5b**, 1.24 mmol, 0.5 g) and quenched after 1 h. The product **6b** was isolated as a white amorphous solid (90 mg, 25 %).

(6b): R*_{f}* = 0.4 [DCM:MeOH (9:1)]; ¹H NMR (400 MHz, MeOD-*d₄*) δ 8.61 (d, *J* = 5.8 Hz, 1H), 5.80 (t, *J* = 1.8 Hz, 1H), 4.21 - 4.11 (m, 2H), 4.08 (dt, *J* = 7.1, 2.4 Hz, 1H), 3.98 (dd, *J* = 12.4, 2.4 Hz, 1H), 3.80 (dd, *J* = 12.4, 2.3 Hz, 1H), 2.56 (s, 3H); ¹³C NMR (100 MHz, MeOD-*d₄*) δ 171.3 (d, *J* = 17.6 Hz), 154.6, 145.7 (d, *J* = 240.0 Hz), 127.1 (d, *J* = 35.4 Hz), 92.9, 85.8, 76.6, 69.6, 60.8, 12.0 (d, *J* = 2.2 Hz); HRMS (ESI) calcd for C₁₀H₁₄O₅N₂SF (M+H)⁺: 293.0602; found 293.0603.

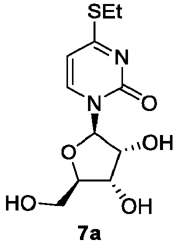

### 4-S-Ethylthiouridine (7a)

4-*S*-Ethylthiouridine (**7a**) was synthesized following the procedure for 4-*S*-methylthiouridine (**6a**). The reaction was done using 2',3',5'-tri-O-acetyl-4-thiouridine (**5a**, 1.29 mmol, 0.5 g) and iodoethane (4.2 mmol, 335 µL). It was quenched after 18 h. The product **7a** was isolated as a white amorphous solid (310 mg, 83 %).

(**7a**): R*_{f}* = 0.4 [DCM:MeOH (9:1)]; ¹H NMR (400 MHz, MeOD-*d₄*) δ 8.31 (d, *J* = 7.1 Hz, 1H), 6.43 (d, *J* = 7.2 Hz, 1H), 5.85 (d, *J* = 1.9 Hz, 1H), 4.18 - 4.11 (m, 2H), 4.12 - 4.03 (m, 1H), 3.94 (dd, *J* = 12.4, 2.5 Hz, 1H), 3.78 (dd, *J* = 12.4, 2.9 Hz, 1H), 3.17 (q, *J* = 7.3 Hz, 2H), 1.34 (t, *J* = 7.4 Hz, 3H); ¹³C NMR (100 MHz, MeOD-*d₄*) δ 180.0, 156.4, 142.1, 105.2, 93.0, 85.8, 76.5, 70.1, 61.4, 25.0, 14.6.

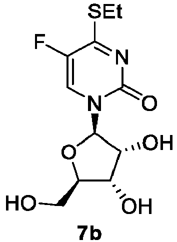

### 5-Fluoro-4-S-ethylthiouridine (7b)

5-Fluoro-4-S-ethylthiouridine **(7b**) was synthesized following the procedure for 4-S-methylthiouridine (**6a**). The reaction was done using 2',3',5'-tri-O-acetyl-5-fluoro-4-thiouridine (**5b**, 1.24 mmol, 0.5 g) and iodoethane (2.5 mmol, 200 µL). It was quenched after 2 h. The product **7b** was isolated as a white amorphous solid (150 mg, 39 %).

(**7b**): R*_{f}* = 0.5 [DCM:MeOH (9:1)]; ¹H NMR (400 MHz, MeOD-*d₄*) δ 8.61 (d, *J* = 5.8 Hz, 1H), 5.80 (t, *J* = 1.7 Hz, 1H), 4.21 - 4.11 (m, 2H), 4.08 (dt, *J* = 7.0, 2.3 Hz, 1H), 3.98 (dd, *J* = 12.4, 2.4 Hz, 1H), 3.79 (dd, *J* = 12.4, 2.3 Hz, 1H), 3.22 (q, *J* = 7.4 Hz, 2H), 1.37 (t, *J* = 7.4 Hz, 3H); ¹³C NMR (100 MHz, MeOD-*d₄*) δ 170.9 (d, *J =* 17.4 Hz), 154.6, 145.5 (d, *J* = 239.9 Hz), 127.3 (d, *J* = 35.4 Hz), 92.9, 85.8, 76.6, 69.7, 60.8, 24.3 (d, *J* = 1.9 Hz), 14.4; HRMS (ESI) calculated for C₁₁H₁₆O₅N₂SF: 307.0758; found 307.0760.

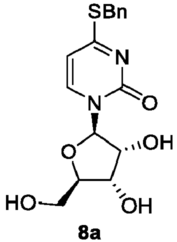

### 4-S-Benzylthiouridine (8a)

4-S-Benzylthiouridine (**8a**) was synthesized following the procedure for 4-*S*-methylthiouridine (**6a**). The reaction was done using 2',3',5'-tri-O-acetyl-4-thiouridine (**5a**, 3.1 mmol, 1.18 g) and benzylbromide (3.4 mmol, 410 µL). It was quenched after 20 min. The product 8a was isolated as a white amorphous solid (700 mg, 64 %).

(**8a**): R*_{f}* = 0.4 [DCM:MeOH (9:1)]; ¹H NMR (400 MHz, MeOD-*d₄*) δ 8.34 (d, *J* = 7.1 Hz, 1H), 7.44 - 7.38 (m, 2H), 7.34 - 7.19 (m, 3H), 6.43 (d, *J* = 7.2 Hz, 1H), 5.87 (d, *J* = 2.1 Hz, 1H), 4.44 (s, 2H), 4.18 - 4.11 (m, 2H), 4.08 (dt, *J* = 5.9, 2.7 Hz, 1H), 3.94 (dd, *J* = 12.4, 2.4 Hz, 1H), 3.78 (dd, *J* = 12.5, 2.8 Hz, 1H); 13C NMR (100 MHz, MeOD-*d₄*) δ 179.2, 156.3, 142.5, 138.3, 130.2 (2C), 129.6 (2C), 128.5, 104.8, 93.0, 85.9, 76.5, 70.1, 61.4, 34.6; HRMS (ESI) calculated for C₁₆H₁₉O₅N₂S (M+H)⁺: 351.1009; found 351.1007.

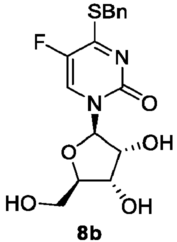

### 5-Fluoro-4-S-benzylthiouridine (8b)

5-Fluoro-4-*S*-benzylthiouridine (**8b**) was synthesized following the procedure for 4-S-methylthiouridine (**6a**). The reaction was done using 2',3',5'-tri-O-acetyl-5-fluoro-4-thiouridine (**5b**, 1.24 mmol, 0.5 g) and benzylbromide (2.0 mmol, 240 µL). It was quenched after 2 h. The product 8b was isolated as a white amorphous solid (130 mg, 28 %).

(**8b**): R*_{f}* = 0.5 [DCM:MeOH (9:1)]; ¹H NMR (400 MHz, MeOD-*d₄*) δ 8.64 (d, *J* = 5.7 Hz, 1H), 7.47 - 7.39 (m, 2H), 7.35 - 7.20 (m, 3H), 5.82 (t, *J* = 1.6 Hz, 1H), 4.48 (s, 2H), 4.21 - 4.12 (m, 2H), 4.08 (dt, *J* = 6.7, 2.4 Hz, 1H), 3.98 (dd, *J* = 12.4, 2.4 Hz, 1H), 3.79 (dd, *J* = 12.5, 2.3 Hz, 1H); ¹³C NMR (100 MHz, MeOD-*d₄*) δ 170.1 (d, *J* = 17.9 Hz), 154.4, 145.2 (d, *J* = 239.7 Hz), 137.8, 130.3 (2C), 129.7 (2C), 128.7, 127.7 (d, *J* = 35.5 Hz), 93.0, 85.8, 76.6, 69.6, 60.8, 33.8 (d, *J* = 1.8 Hz; HRMS (ESI) calculated for C₁₆H₁₈O₅N₂SF (M+H)⁺: 369.0919; found 369.0919.

### Example 2. Synthesis of N⁴-alkylated 2'-deoxycytidine derivatives

**General information.** Chemicals and solvents were purchased from Sigma-Aldrich and Alfa Aesar and used without further purification. Thin-layer chromatography (TLC) was carried out on 25 TLC aluminium sheets coated with silica gel 60 F₂₅₄ (Merck) and column chromatography on silica gel 60 (0.063-0.200 nm) (Merck). Reverse phase chromatography was carried out on Grace flash cartridges C-18. NMR spectra were recorded in DMSO-d₆ on a Bruker Ascend 400 ¹H NMR-400 MHz, ¹³C NMR - 101 MHz. Chemical shifts are reported in ppm relative to the solvent resonance signal as an internal standard. HPLC-MS analyses were performed using a high performance liquid chromatography system, equipped with a photo diode array detector (SPD-M20A) and a mass spectrometer (LCMS-2020, Shimadzu, Japan) equipped with an ESI source. The chromatographic separation was conducted using a YMC Pack Pro column, 3×150 mm (YMC, Japan) at 40 °C and a mobile phase that consisted of 0.1 % formic acid water solution (solvent A), and acetonitrile (solvent B). Mass spectrometry data was acquired in both positive and negative ionization mode and analysed using the LabSolutions LCMS software.

### General procedure for the synthesis of N⁴-alkylated 2'-deoxycytidines

The alkylation reactions of 4-thio-uridines were prepared by modifying methods reported in literature.

To a solution of 4-thio-2'-deoxyuridine (37 mg, 0.15 mmol) in 70 % aqueous ethanol solution (1 mL) appropriate amine (0.45 mmol) was added. The mixture was stirred for 24-60 h at 55 °C temperature. Completion of the reaction was determined by thin-layer chromatography (TLC, chloroform/methanol, 9/1). After the reaction was completed (TLC), solvents were evaporated under reduced pressure. The residue was dissolved in either chloroform and purified by column chromatography (silica gel, chloroform/methanol mixture, 10:0→9:1) or water and purified by reverse phase column chromatography (C-18 cartridges, water/methanol mixture, 10:0→10:2). After purification, the solvents were removed under reduced pressure to afford *N*⁴-alkylated-2'-deoxycytidines in 53-91 % yield. Synthesized modified nucleosides were characterized by NMR spectroscopy and HPLC-MS analysis.

### General procedure for the synthesis of 5-fluoro- N⁴-alkylated -2'-deoxycytidines

To a solution of 5-fluoro-4-thio-2'-deoxyuridine (39 mg, 0.15 mmol) in 75 % aqueous ethanol solution (1 mL) appropriate amine (0.45 mmol) was added. The mixture was stirred for 72-120 h at 55 °C temperature. Completion of the reaction was determined by thin-layer chromatography (TLC, chloroform/methanol, 9/1). After the reaction was completed (TLC), solvents were evaporated under reduced pressure. The residue was dissolved in either chloroform and purified by column chromatography (silica gel, chloroform/methanol mixture, 10:0→9:1) or water and purified by reverse phase column chromatography (C-18 cartridges, water/methanol mixture, 10:0→10:2). After purification, the solvents were removed under reduced pressure to afford *N*⁴-alkylated-5-fluoro-2'-deoxycytidines in 39-81 % yield. Synthesized modified nucleosides were characterized by NMR spectroscopy and HPLC-MS analysis.

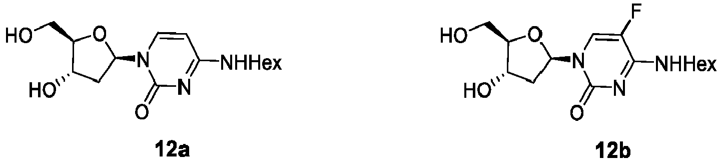

***N*⁴-hexyl-2'-deoxycytidine (12a).** Yield 42 mg (90 %).

MS (ESI⁺): m/z 312.00 [M+H]⁺, 310.00 [M-H]⁻. UV (CH₃OH) λₘₐₓ 282 nm.

¹H NMR (400 MHz, DMSO-d₆): δ = 7.65 (d, *J* = 7.5 Hz, 1H, CH=), 7.61 (t, *J* = 5.5 Hz, 1H, NH), 6.09 (dd, *J* = 7.3, 6.2 Hz, 1H, CH), 5.66 (d, *J* = 7.5 Hz, 1H, CH=), 5.12 (d, *J* = 4.2 Hz, 1H, OH), 4.92 (t, *J* = 10.6, 4.2 Hz, 1H, OH), 4.21-4.06 (m, 1H, CH), 3.76-3.61 (m, 1H, CH), 3.55-3.38 (m, 2H, CH₂), 3.22-3.07 (m, 2H, CH₂), 2.11-1.95 (m, 1H, CH₂), 1.93-1.79 (m, 1H, CH₂), 1.45-1.35 (m, 2H, CH₂), 1.25-1.17 (m, 6H, CH₂), 0.79 (t, *J* = 4.8 Hz, 3H, CH₃).

¹³C NMR (101 MHz, DMSO-d₆): δ = 163.70, 155.56, 140.11, 95.12, 87.60, 85.25, 70.92, 61.88, 40.73, 31.49, 29.01, 26.63, 22.54, 14.38.

**5-fluoro-*N*⁴-hexyl-2'-deoxycytidine (12b).** Yield 40 mg (81 %).

MS (ESI⁺): *m*/*z* 329.85 [M+H]⁺, 327.85 [M-H]⁻. UV (CH₃OH) λₘₐₓ 280; 250 nm.

¹H NMR (400 MHz, DMSO-d₆): δ = 8.05 (d, *J* = 7.4 Hz, 1H, CH=), 8.03-7.99 (m, 1H, NH), 6.12 (t, *J* = 7.4 Hz, 1H, CH), 5.21 (d, *J* = 4.2 Hz, 1H, OH), 5.10 (t, *J* = 5.1 Hz, 1H, OH), 4.26-4.16 (m, 1H, CH), 3.95-3.71 (m, 1H, CH), 3.67-3.49 (m, 2H, CH₂), 3.28 (dd, *J* = 13.5, 6.6 Hz, 2H,CH₂), 2.11 (ddd, *J* = 13.1, 5.9, 3.5 Hz, 1H,CH₂), 1.98 (dt, *J* = 13.1, 6.4 Hz, 1H, CH₂), 1.60-1.43 (m, 2H,CH₂), 1.35-1.18 (m, 6H, CH₂), 0.86 (t, *J*= 6.7 Hz, 3H,CH₃).

¹³C NMR (101 MHz, DMSO-d₆): δ = 155.31, 153.76, 138.00, 135.59, 124.55, 87.77, 85.57, 70.63, 61.55, 40.84, 31.45, 28.80, 26.55, 22.53, 14.35.

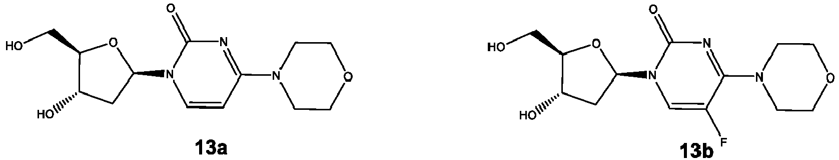

**4-(4-morpholinyl)-2'-deoxyuridine** (**13a**). Yield 36 mg (81 %).

MS (ESI⁺): *m*/*z* 297.90 [M+H]⁺, 295.90 [M-H]⁻. UV (CH₃OH) λₘₐₓ 286 nm.

¹H NMR (400 MHz, DMSO-d₆): δ = 7.96 (d, *J* = 7.8 Hz, 1H, CH=), 6.18 (d, *J* = 7.8 Hz, 1H, CH=), 6.14 (t, *J* = 6.6 Hz, 1H, CH), 5.22 (bs, 1H, OH), 5.04 (bs, 1H, OH), 4.27-4.17 (m, 1H, CH), 3.84-3.76 (m, 1H, CH), 3.68-3.54 (m, 10H, 5CH₂), 2.21-2.10 (m, 1H, CH₂), 2.01-1.91 (m, 1H, CH₂).

¹³C NMR (101 MHz, DMSO-d₆): δ = 163.06, 154.83, 142.15, 91.60, 87.79, 85.52, 70.72, 66.18, 65.99, 61.67, 41.01.

**5-fluoro-4-(4-morpholinyl)-2'-deoxyuridine (13b).** Yield 26 mg (55 %).

MS (ESI⁺): *m*/*z* 315.85 [M+H]⁺. UV (CH₃OH) λₘₐₓ 293 nm.

¹H NMR (400 MHz, DMSO-d₆): δ = 8.22 (d, *J* = 10.2 Hz, 1H, CH=), 6.07 (t, *J* = 6.4 Hz, 1H,CH), 5.22 (bs, 2H, 2OH), 4.30-4.18 (m, 1H, CH), 3.81-3.76 (m, 1H, CH), 3.73-3.70 (m, 2H,CH₂), 3.68-3.64 (m, 4H,CH₂), 3.62- 3.56 (m, 4H,CH₂), 2.18-2.11 (m, 1H,CH₂), 2.04-1.97 (m, 1H,CH₂).

¹³C NMR (101 MHz, DMSO-d₆): δ = 154.45, 152.69, 138.44, 136.01, 128.73, 87.93, 85.66, 70.43, 66.44, 61.35, 46.41, 45.38, 41.01.

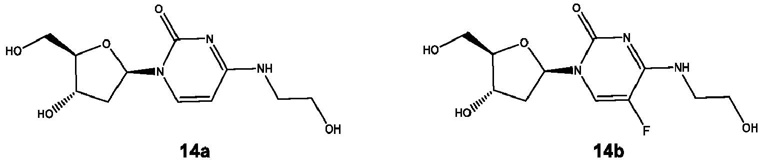

***N*⁴-2-hydroxyethyl-2'-deoxycytidine (14a).** Yield 30 mg (75 %).

MS (ESI⁺): *m*/*z* 271.90 [M+H]⁺, 269.90 [M-H]⁻. UV (CH₃OH) λₘₐₓ 281 nm.

¹H NMR (400 MHz, DMSO-d₆): δ = 7.79 (t, *J* = 5.5 Hz, 1H, NH), 7.73 (d, *J* = 7.5 Hz, 1H, CH=), 6.16 (dd, *J* = 10.3, 3.1 Hz, 1H, CH), 5.80 (d, *J* = 7.5 Hz, 1H, CH=), 4.19 (dt, *J* = 6.1, 3.1 Hz, 1H, CH), 3.77-3.74 (m, 1H, CH), 3.57-3.52 (m, 2H, CH₂), 3.49 (d, *J* = 5.8 Hz, 2H, CH₂), 3.31 (q, *J* = 5.6 Hz, 2H, CH₂), 2.17-2.02 (m, 1H, CH₂), 1.93 (dd, *J* = 13.9, 6.7 Hz, 1H, CH₂).

¹³C NMR (101 MHz, DMSO-d₆): δ = 163.96, 155.51, 140.18, 95.22, 87.61, 85.27, 70.87, 61.84, 59.96, 43.11, 40.75.

**5-fluoro-*N*⁴-2-hydroxyethyl-2'-deoxycytidine (14b).** Yield 32 mg (74 %).

MS (ESI⁺): *m*/*z* 289.80 [M+H]⁺, 287.80 [M-H]⁻. UV (CH₃OH) λₘₐₓ 280; 245 nm.

¹H NMR (400 MHz, DMSO-d₆): δ = 8.06 (d, *J* = 7.3 Hz, 1H, CH=), 7.94 (s, 1H, NH), 6.11 (t, *J* = 6.2 Hz, 1H, CH), 5.12 (bs, 2H, 2OH), 4.79 (bs, 1H, OH), 4.26-4.16 (m, 1H, CH), 3.81-3.74 (m, 1H, CH), 3.66-3.56 (m, 2H, CH₂), 3.53-3.49 (m, 2H, CH₂), 3.45-3.40 (m, 2H), 2.18-2.05 (m, 1H, CH₂), 1.98 (dt, *J* = 13.1, 6.5 Hz, 1H, CH₂).

¹³C NMR (101 MHz, DMSO-d₆): δ = 155.63, 153.67, 138.03, 135.64, 124.74, 87.74, 85.58, 70.61, 61.54, 59.49, 42.94.

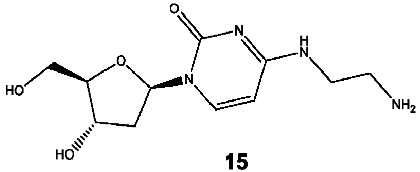

***N*⁴-2-aminoethyl-2'-deoxycytidine** (**15**). Yield 37 mg (91 %).

MS (ESI⁺): *m*/*z* 270.95 [M+H]⁺, 268.95 [M-H]⁻. UV (CH₃OH) λₘₐₓ 273 nm.

¹H NMR (400 MHz, DMSO-d₆): δ = 8.01-7.76 (m, 1H, NH), 7.74 (d, *J* = 7.5 Hz, 1H, CH=), 6.21-6.10 (m, 1H, CH), 5.77 (d, *J* = 7.5 Hz, 1H, CH=), 4.20 (dt, *J* = 6.1, 3.2 Hz, 1H, CH), 3.80-3.71 (m, 3H, CH, CH₂), 3.60-3.48 (m, 2H, CH₂), 3.25 (s, 2H, CH₂), 2.67 (t, *J* = 6.3 Hz, 2H, CH₂), 2.15-2.02 (m, 1H, CH₂), 1.98-1.88 (m, 1H, CH₂).

¹³C NMR (101 MHz, DMSO-d₆): δ = 163.91, 155.53, 140.08, 95.19, 87.62, 85.26, 70.84, 61.81, 43.46, 41.06, 40.73.

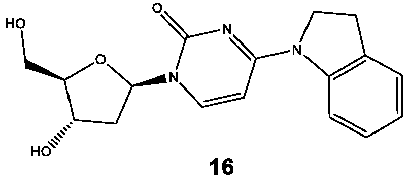

**4-(indolin-1-yl)-2'-deoxyuridine** (**16**). Yield 26 mg (53 %).

MS (ESI⁺): *m*/*z* 329.90 [M+H]⁺, 327.90 [M-H]⁻. UV (CH₃OH) λₘₐₓ 320 nm.

¹H NMR (400 MHz, DMSO-d₆): δ = 8.54 (s, 2H), 8.13 (d, *J* = 7.6 Hz, 2H), 7.26 (d, *J* = 7.3 Hz, 2H), 7.20 (t, *J* = 7.7 Hz, 2H), 7.00 (td, *J* = 7.4, 0.8 Hz, 2H), 6.20 (t, *J* = 6.5 Hz, 2H), 6.16 (d, *J* = 7.3 Hz, 1H), 5.25 (d, *J* = 4.2 Hz, 2H), 5.06 (t, *J* = 5.2 Hz, 2H), 4.24 (td, *J* = 7.3, 3.6 Hz, 2H), 4.10 (t, *J* = 8.7 Hz, 2H), 3.83 (q, *J* = 3.6 Hz, 2H), 3.67-3.51 (m, 5H), 3.19 (t, *J* = 8.5 Hz, 2H), 2.28-2.16 (m, J= 13.2, 6.0, 3.6 Hz, 2H), 2.07-1.95 (m, 2H).

¹³C NMR (101 MHz, DMSO-d₆): δ = 161.21 (s), 154.06 (s), 143.23 (s), 142.36 (s), 133.14 (s), 127.07 (s), 125.17 (s), 123.50 (s), 94.03 (s), 87.97 (s), 85.90 (s), 70.69 (s), 61.58 (s), 49.01 (s), 40.62 (s), 27.22 (s).

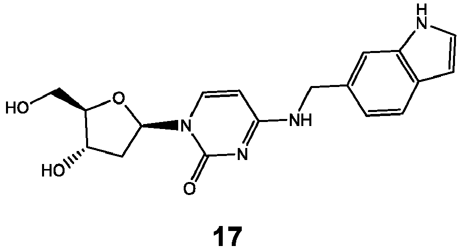

***N*⁴-((1H-indol-6-yl)methyl)-2'-deoxycytidine** (17). Yield 35 mg (65 %).

MS (ESI⁺): m/z 357.00 [M+H]⁺, 355.00 [M-H]⁻. UV (CH₃OH) λₘₐₓ 282 nm.

¹H NMR (400 MHz, DMSO-d₆): δ = 11.05 (s, 1H, NH), 8.13 (t, *J* = 5.7 Hz, 1H, NH), 7.77 (d, *J* = 7.5 Hz, 1H, CH=), 7.48 (d, *J* = 8.1 Hz, 1H, CH), 7.32 (s, 1H), 7.31-7.29 (m, 1H, CH), 6.95 (dd, J= 8.1, 1.3 Hz, 1H, CH), 6.41-6.36 (m, 1H, CH), 6.22-6.15 (m, 1H, CH), 5.83 (d, *J* = 7.5 Hz, 1H, CH=), 5.20 (d, *J* = 4.2 Hz, 1H, OH), 4.97 (t, *J* = 5.3 Hz, 1H, OH), 4.56 (d, *J* = 5.7 Hz, 2H, CH₂), 4.28-4.14 (m, *J* = 3.3 Hz, 1H, CH), 3.83-3.66 (m, 1H, CH), 3.61-3.48 (m, 2H, CH₂), 2.15 - 2.05 (m, 1H, CH₂), 2.00-1.90 (m, 1H, CH₂).

¹³C NMR (101 MHz, DMSO-d₆): δ = 163.59, 155.53, 140.31, 136.48, 132.02, 127.20, 125.80, 120.24, 110.78, 101.33, 95.10, 87.64, 85.24, 79.70, 70.88, 61.82, 56.54, 44.18.

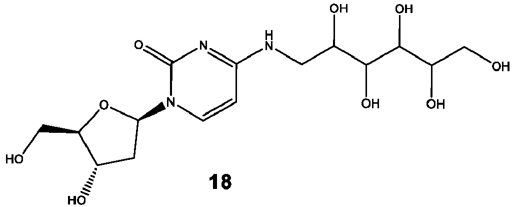

***N*⁴-(2,3,4,5,6-pentahydroxyhexyl)-2'-deoxycytidine** (**18**). Yield 35 mg (60 %).

MS (ESI⁺): *m*/*z* 391.85 [M+H]⁺. UV (CH₃OH) λₘₐₓ 282 nm.

¹H NMR (400 MHz, DMSO-d₆): δ = 7.73 (d, J= 7.5 Hz, 1H, CH=), 7.70 (t, *J* = 5.6 Hz, 1H, NH), 6.18-6.13 (m, 1H, CH), 5.85 (d, *J* = 7.5 Hz, 1H, CH=), 5.19 (d, *J* = 4.1 Hz, 1H, OH), 5.08-4.85 (m, 2H, 2OH), 4.49 (d, *J* = 5.2 Hz, 1H, OH), 4.43 (t, *J* = 5.8 Hz, 2H, CH₂), 4.36 (t, *J* = 5.4 Hz, 1H, CH), 4.22-4.16 (m, 1H, CH), 3.76 (dd, *J* = 6.9, 3.7 Hz, 1H, CH), 3.73- 3.65 (m, 1H, CH), 3.62-3.51 (m, 4H, CH₂, OH), 3.52-3.43 (m, 3H, CH₂, OH), 3.40 (dd, *J* = 9.5, 4.0 Hz, 1H, CH), 3.29-3.13 (m, 1H, CH), 2.09 (ddd, *J* = 13.1, 5.9, 3.6 Hz, 1H, CH₂), 2.02-1.83 (m, 1H, CH₂).

¹³C NMR (101 MHz, DMSO-d₆): δ = 164.05, 155.38, 140.09, 95.27, 87.53, 85.21, 72.54, 72.08, 71.95, 70.89, 70.06, 63.78, 61.84, 43.66, 40.72.

### Synthesis of 5-fluoro-N⁴-((1H-indol-3-yl)propionyl)-cytidine (19)

Indole propionic acid was activated in the mixture of indole propionic acid (1 mmol), *N*,*N-*dicyclohexylcarbodiimide (DCC) (1mmol) and *N*-hydroxysuccinimide (NHS (1mmol) in 10 ml solution of ethylacetate. The mixture was stirred for 24h at room temperature (25-30°C). 5-fluoro-cytidine (261 mg, 1 mmol) solution in *N*,*N*-dimethylformamide (1ml) was added to a activated acid solution. The mixture was stirred for 72h at room temperature (25-30°C). Completion of the reaction was determined by thin-layer chromatography (TLC, chloroform/methanol, 9/1). After the reaction was completed (TLC), solvents were evaporated under reduced pressure. The residue was dissolved in either chloroform and purified by column chromatography (silica gel, chloroform/methanol mixture, 2%→16% gradient of methanol). Synthesized modified nucleoside were characterized by NMR spectroscopy and HPLC-MS analysis.

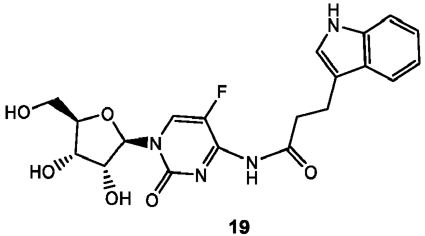

**5-fluoro-*N*⁴-((1*H*-indol-3-yl)propionyl)-cytidine (19).** Yield 35 mg (60 %).

HPLC-MS (ESI+): m/z 433 [M+H]⁺; 431 [M-H]⁻. UV - λₘₐₓ₁ = 247 nm, λₘₐₓ₂ = 291 nm.

¹H (DMSO-*d₆*, 400 MHz) δ = 2.85-2.94 (2H, m, -CH₂); 2.95-3.04 (2H, m, -CH₂); 3.66-3.55 (1H, m, -CH); 3.72-3.89 (1H, m, -CH); 3.87-3.94 (1H, m, -CH); 3.99 (2H, s, -CH₂); 4.11 (1H, d, *J* = 5.4 Hz, -OH); 5.36 (1H, d, *J* = 4.5 Hz, -OH); 5.53-5.59 (1H, m, -OH); 5.67-5.76 (1H, m, -CH); 6.95-7.01 (1H, m, -CH); 7.03-7.10 (1H, m,-CH); 7.09-7.14 (1H, m, -CH); 7.13 (1H, d, *J* = 2.3 Hz, -CH); 7.33 (1H, d, *J* = 8.1 Hz, =CH); 7.57 (1H, d, *J* = 7.7 Hz, =CH); 8.70 (1H, d, *J* = 6.8 Hz, -CH); 10.72-10.86 (1H, m, -NH). ¹³C BMR (DMSO-*d₆*, 101 MHz) δ = 20.60; 25.69; 25.92; 37.88; 49.07; 59.85; 60.59; 68.67; 69.45; 74.69; 74.99; 84.53; 89.73; 90.79; 111.79; 113.77; 118.66; 118.83; 121.39; 121.39; 122.83; 123.17; 127.43; 136.69; 170.72; 173.24.

### Example 3. Identifying hydrolases genes and cloning them into E. coli expression vector

The clones exhibiting hydrolase activity were identified on a MD medium (33.9 g/L Na₂HPO₄ 15 g/L KH₂PO₄, 5 g/L NH₄Cl, 2.5 g/L NaCl, 0.2% (w/v) glucose, 0.2% Casamino acid, 1 mM IPTG) containing 100 µg/mL ampicillin, 0.02 mg/mL *N*⁴-benzoyl-2'-deoxycytidine (selected Seq ID NO: 1/ 2/ 3 ) or *S*⁴- -methylthiouridine (selected SEQ ID NO: 25/ 26/ 27), or *S*⁴-ethylthiouridine (selected SEQ ID NO: 28/29/30/31), or containing mix of substrates *S*⁴-methylthiouridine, *S*⁴-ethylthiouridine and *S*⁴-benzylthiouridine (SEQ ID NO: 32 - 40) as the sole source of uridine, allowing only the growth of recombinants that can complement the uridine auxotrophy of the *E. coli* DH10B *ΔpyrFEC*::Km (A. Aučynaitė et. al., 2018) strain by hydrolysing cytidine derivatives. Metagenomic hydrolases encoding genes were amplified with Phusion DNA polymerase using forward (**Fw**) and reverse primers (**Rv**) by using standard PCR protocol (ThermoFisher Scientific) **Fw/Rv**:

AGAAGGAGATATAACTATGATGAACAAGGAAGATTTATTG/GTGGTGGTGATGGTGATGGC
CTAAAAGATCCTCTTTCGTGA (SEQ ID NO: 1);

AGAAGGAGATATAACTATGAGGCATGACCTCGTC/GTGGTGGTGATGGTGATGGCCA-
CCCAAACTGTTCGCGTC (SEQ ID NO: 2);

AGAAGGAGATATAACTATGAAGGAAACACTTT-
GTCA/GTGGTGGTGATGGTGATGGCCTTTTCCGTAATCCTCGCTC (SEQ ID NO: 3);

AGAAGGAGATATAACTATGAACAGACAAGAATTAATAACGTGGTGGTGATGGTGATGGCC
AAGCTTTCGTTCGTCATGTAA (SEQ ID NO: 9);

AGAAGGAGATATAACTATGATATTATTGAGCGAAGCT/GTGGTGGTGATGGTGATGGCCCT
CATCAGTTAAAGTAAATCG (SEQ ID NO: 25);

AGAAGGAGATATAACTATGGTAACTAAGAATATTTCC/GTGGTGGTGATGGTGATGGCCCA
GAGGCTTGATCTTCAG (SEQ ID NO: 26);

AGAAGGAGATATAACTATGAGCGCGGAC-
GAAAG/GTGGTGGTGATGGTGATGGCCTTTATCCAGATCTCCCGG (SEQ ID NO: 27);

AGAAGGAGATATAACTATGAAAAAGATATCAATTACAAC/GTGGTGGTGATGGTGATGGCC
CTCATCCGAAGAGCTAAG (SEQ ID NO: 28);

AGAAGGAGATATAACTATGAG-
TAAGGTTTCTGATCAGG,GTGGTGGTGATGGTGATGGCCTGCCAGATCCTTCGGTC
(SEQ ID NO: 29);

AGAAGGAGATATAACTATGACCGACAGAC-
GCATCA/GTGGTGGTGATGGTGATGGCCTTCCACTTTCAAACTGTCGA (SEQ ID NO: 30);

AGAAGGAGATATAACTATGCGGACCAAAGAAGATT/GTGGTGGTGATGGTGATGGCCGTC
GAGCAGGCGGAAAG (SEQ ID NO: 31);

AGAAGGAGATATAAC-
TATGACAGATTGGACATATTTGAT/GTGGTGGTGATGGTGATGGCCTTTTGCAAGATCTTC
TGC (SEQ ID NO: 32);

AGAAGGAGATATAACTATGGAC-
CTCGAGCGC/GTGGTGGTGATGGTGATGGCCCTTCTTCGCCATATAGTTC (SEQ ID NO:
33);

AGAAGGAGATATAACTATGACCGAAAAGAAAATCACC/GTGGTGGTGATGGTGATGGCCA
AACTCGGTGAAGGAAAG (SEQ ID NO: 34);

AGAAGGAGATATAACTATGAACCAACTGACGCAAC/GTGGTGGTGATGGTGATGGCCGA
CCCCCAGGTCCTTGG (SEQ ID NO: 35);

AGAAGGAGATATAACTATGACCAATGTCAC-
TCCCG/GTGGTGGTGATGGTGATGGCCGGATTTTAGATGCGCGGG (SEQ ID NO: 36);

AGAAGGAGATATAACTATGACAGAGACAGACAAGAAG/GTGGTGGTGATGGTGATGGCC
TCCGTTAATGTCCTCGGC (SEQ ID NO: 37);

AGAAGGAGATATAAC-
TATGGATGCAGGTTTGTTGATG/GTGGTGGTGATGGTGATGGCCCTGCTTGTTTAAATCC
CATG (SEQ ID NO: 38).

SEQ ID NO: 4 - 8 genes were synthesised at Twist Bioscience, USA South San Francisco. All genes of hydrolases were ligated into pLATE31 (Thermo Fisher Scientific) expression vectors by using aLICator LIC Cloning and Expression Kit 3 protocol (Thermo Fisher Scientific). It was prepared the following reaction mixture at room temperature: 5X LIC Buffer 2 µL, Purified PCR product 0.1 pmol, water, nuclease-free to 9 µL and T4 DNA Polymerase (1u/µL) 1 µL. Total mix volume was 10 µL. The reaction mixture was incubated at room temperature (20-25°C) for 5 min. after incubation the reaction was stopped by adding 0.6 µL of 0.5M EDTA. Than1 µL pLATE31, LIC-ready vector (60 ng, 0.02 pmol DNA) was added. The annealing mixture was incubated at room temperature (20-25°C) for 30 min. Transformation of competent DH5α *E.coli* cells was performed by electroporation. Finally, the cells were plated on LB agar supplemented with relative antibiotics. Plasmid DNA was extracted using "ZR Plasmid Miniprep - Classic" (Zymo Research) and sequenced.

### Example 4. Site-directed Mutagenesis of genes

Mutations in the hydrolase's genes were introduced using methodology based on "Phusion^{™} Site-Directed Mutagenesis Kit" (Thermo Fisher Scientific). For specific mutagenesis, primer pairs meant to amplify the whole plasmid with the target protein gene, where one primer has miss-matched base pairs in the middle, were created (**Fw/Rv**):
CTTTTAGGCCATCACCATCACCAC/GAATCGCATCGGCAACAATTCATC (SEQ ID NO:10); 
GATGCGAGCAACGAAAGAGGATCTTTTAG/GGCAACAATTCATCGATCGTTTTTTCTAAC
G (SEQ ID NO:11);
 
GATGCGATGGACGAAAGAGGATCTTTTAG/GGCAACAATTCATCGATCGTTTTTTCTAAC
G (SEQ ID NO:12);
 TGATAGGGTCATCGCACCTTGCG/CCATCGGTTACGATCGCCAACG (SEQ ID NO:13); 
TTACGGAGCGGGTAATTGCGG/GAAGCATTTTCGATATTTGCGCCTAAAAAC (SEQ ID
NO:14);
 
ATTGCGGTGAATTAAGTGCCATTTTCGC/TGGTCGCTCCGTAAGAAGCATTTTCG (SEQ
ID NO:15);
 GCACCTTGCGGAATCTGCC/ATCACCATCGGTTACGATCGCC and NNNNNNNNNGCACCTTGCGGAATCTGCC/ATCACCATCGGTTACGATCGCC (SEQ ID NO: 16, SEQ ID NO: 17, SEQ ID NO: 18); 
NNNNNNNNNNNNCTTTTAGGCCATCACCATCACCAC/GAATCGCATCGGCAACAATTCA
TC (SEQ ID NO: 19, SEQ ID NO: 20);
 TAACGCGGACATCGCGCCGTG/CCGTTGCAAACAACCGCCAGCG (SEQ ID NO: 21); 
CGGTCTGACCAACTGCGCGG/TAAGACGCGTTTTCAACGTTGCAACCG (SEQ ID NO:
22);
 GAAGGTCCGCTGTCTCCGTGC/GGTGTCCGCCGCAACCG (SEQ ID NO: 23);
AAAAACCGGCGTTCCCGTGCG/CCGGCGCGATAGAAGAAGAGATCGC (SEQ ID NO: 24).

The base pair miss-matches result in coded amino acid changes after PCR. Before PCR the primers were phosphorylated in the reaction mixture: 1.25 µL oligonucleotide (100 µM stock), 2.5 µl 10X reaction buffer A for T4 Polynucleotide Kinase, 2.5 µL ATP (10 mM stock), 1 µL T4 Polynucleotide Kinase (10 U/µL) and H2O up to 25 µL. The mixture was incubated at 37 °C for 30 min followed by heat inactivation at 75 °C for 10 min. DNA was amplified in the PCR reaction mixture: 10 µL 2X Phusion Green Hot Start II High-Fidelity PCR Master Mix, 2 µL phosphorylated forward primer (5 µM stock), 2 µL phosphorylated reverse primer (5 µM stock), 0.5 ng plasmid DNA with hydrolase gene, H₂O up to 20 µL. PCR conditions was: Initial denaturation - 30 s 1 cycle at 98°C, denaturation -10 s at 98°C; Annealing* -15 s 65-72°C (*Annealing temperatures were calculated using Thermo Fisher Scientific Tm Calculator), extension - 20 s/kb at 72°C. 25 cycles were repeated; final extension - 5 min at 72°C. After PCR the amplified products were separated in 1% agarose gel by electrophoresis. The correct length fragment was cut from the agarose gel and purified using "GeneJET Gel Extraction Kit" (Thermo Fisher Scientific). The purified fragments were ligated in the reaction mixture: 1 µL 10X T4 DNA Ligase Buffer, 0.5 µL T4 DNA Ligase (5 U/µL), 1 µL PEG 4000, -10 ng plasmid DNA, up to 10 µL H₂O. The fragments were ligated at 4 °C for 18 h. After incubation ligated DNA fragments were transformed into DH5α *E. coli* cells.

For random mutagenesis (SEQ ID NO: 16, SEQ ID NO: 17, SEQ ID NO: 18, SEQ ID NO: 19, SEQ ID NO: 20) of selected regions a two-step mutagenesis was used. In the first step a pair of primes surrounding the region that will be randomized, but not including it, were used to create a deletion of the region of interest. In the second round one of the original primers was modified by adding a random nucleotide overhang that fills the deleted region, to its 5' end and the other primer was left unmodified. After PCR this fills the gap with random nucleotides and thus random amino acids. All the other steps remained the same as above.

Transformation was performed using chemically competent DH5α *E. coli* cells, these were prepared by growing cells in a 50 mL LB medium until the cell density reached OD600≈0.7, then the cells were chilled on ice and centrifuged at 4 °C for 10 min (2700 xg) the medium was removed and the cells were suspended in same volume of 0.1 M CaCl₂ solution and additionally centrifuged under the same conditions for additional four times, after the last centrifugation the cells were suspended in 1 ml of 0.1 M CaCl₂ solution. 5 µL of the ligation mixture were mixed with 95 µL of prepared DH5α *E*. *coli* cells and incubated on ice for 30 min, after that the cells were heat shocked for 2 min at 42 °C in an air thermostat, then 900 µL of SOC medium was added into the mixture and the cells were kept at 37 °C for 30 min, finally the cells were centrifuged and plated on LB agar supplemented with relative antibiotics.

To select correct mutants several colonies were chosen and inoculated into 5 mL of LB medium with relative antibiotic and left to grow at 30 ° C overnight, then their plasmid DNA was extracted using "ZR Plasmid Miniprep - Classic" (Zymo Research) and sequenced to make sure the mutants were correct.

### Example 5. Enzyme activity detection in vivo in E. coli cells

The activities of hydrolases corresponding to SEQ ID NO: 16, SEQ ID NO: 17, SEQ ID NO: 18, SEQ ID NO: 19 and SEQ ID NO: 20, were selected *in vivo* by using uridine auxotrophic *E*. *coli* strain HMS174 Δ*pyr*FΔ*cdd* cells. *E*. *coli* HMS174 Δ*pyr*FΔ*cdd* competent cells were transformed with the pLATE31 plasmids harbouring the appropriate gene and the clones were cultivated on the selective M9 medium agar plate containing 20 µg/ml *N*⁴-benzoyl-2'-deoxycytidine as the sole source of uridine and 100 µg/ml ampicillin. The cytidine deaminase converts *N*⁴-benzoyl-2'-deoxycytidine to 2'-deoxyuridine, upon which the growth phenotype of uridine auxotrophic cells is restored. As a result, colony growth is observed after 1-2 days.

### Example 6. Overexpression of hydrolases in E. coli cells and purification of them

The recombinant proteins were overexpressed in *E. coli* strain HMS174 (DE3) Δ*pyr*FΔ*cdd.* E. *coli* cells were grown in 20 mL BHI (Brain-Heart-Infusion Broth) medium containing ampicillin (100 mg/mL) at 37°C with aeration. Protein expression was induced by adding 0.5 µM IPTG at 0.6-1 OD₆₀₀, and cells were grown for a further 18-20 h at 30°C. Wet cell biomass from 20 mL culture broth were suspended in 5-10 mL of buffer A (50 mM potassium phosphate, pH 7.5), and disrupted by sonication for 2.5 min. A lysate was cleared by centrifugation at 15,000×*g* for 4 min. Cleared lysate was applied to 1 mL Ni-NTA column (equilibrated with the buffer A). The column was washed with the buffer A, and the proteins were eluted with buffer A containing 300 mM imidazole. The active fractions were combined and dialyzed against the buffer B (50 mM potassium phosphate, pH 7.5), at 25 °C. All the purification procedures were performed at room temperature. The active fractions were combined and dialyzed against the buffer A (50 mM potassium phosphate, pH 7.5) with 50% glycerol at 4 °C. The concentration of proteins was determined using Pierce^{™} Coomassie Plus (Bradford) Assay Reagent and the Standard Microplate Protocol. To check the homogeneity, proteins were analysed by sodium dodecyl sulphate polyacrylamide gel electrophoresis (SDS-PAGE, 14% separating and 4.0% stacking gels. Gels were developed in Coomassie Brilliant Blue G-250 dye. Each sample contained 2-3 µg of total protein. Purified proteins were stored at - 20°C.

### Example 7. Analysis of substrate spectrum of hydrolases

A hydrolytic activity of enzymes was analysed by thin-layer chromatography (TLC), high-performance liquid chromatography and a mass spectrometry (HPLC-MS) and Gas chromatography and a mass spectrometry (GC-MS) methods. Nucleosides, as substrates and hydrolysis reaction products, were tested by thin-layer chromatography (TLC) method and by a high-performance liquid chromatography - mass spectroscopy (HPLC-MS) method. Other reaction products were detected by Gas chromatography-Mass spectrometry (GC-MS) system. The analysis results are submitted in the **Table 1, 2, 3, 4.** The conditions of the reactions are given below.

Hydrolysis products test by thin-layer chromatography (TLC) method. A hydrolytic activity was assayed in reaction mixture containing 45 mM potassium phosphate buffer, pH 7.5, 1 µl enzyme (0.1-4.6 µg/reaction) and 4 mM substrate. The total reaction mix volume was 20 µl. Reaction mixture were incubated at 30 °C temperature up to 3 h and at RT overnight. Thin-layer chromatography (TLC) was conducted on the Merck silica gel 60 F254 plates, using the chloroform and methanol (5:1) mixture of solvents.

High-performance liquid chromatography/mass spectrometry (HPLC-MS) analysis of hydrolysis reaction products. HPLC-MS analyses were performed using a high-performance liquid chromatography system (Shimadzu, Japan) equipped with a photo diode array (PDA) detector (SPD-M20A Prominence diode array detector; Shimadzu, Japan) and a mass spectrometer (LCMS-2020, Shimadzu, Japan) equipped with an ESI source. The chromatographic separation was conducted using a Hydrosphere C18 column, 4 × 150 mm (YMC, Japan), or YMC Pack Pro column, 3× 150 mm (YMC, Japan) at 40 °C and a mobile phase that consisted of 0.1 % formic acid water solution (solvent A), and acetonitrile (solvent B) delivered in the gradient elution mode. Mass scans were measured from m/z 10 up to m/z 500, at 350 °C interface temperature, 250 °C DL temperature, ±4,500 V interface voltage, neutral DL/Qarray, using N 2 as nebulizing and drying gas. Mass spectrometry data was acquired in both the positive and negative ionization mode. The data was analysed using the LabSolutions LCMS software. For HPLC-MS analysis, the reaction mixture consisted of 45 mM potassium phosphate buffer (pH 7.5), 2 mM substrate (from 50-100 mM stock in DMSO), and protein (-0.5-1 mg/ ml). Reaction mixture was incubated at 30 °C temperature up to 3 h and at RT overnight.

Gas chromatography-Mass spectrometry (GC-MS) analysis of hydrolysis reaction products. GC-MS analyses were performed with a Shimadzu GCMS-QP2010 Ultra Plus (Kyoto, Japan). Chromatographic separation was achieved on a Rxi^{®}-5ms column (30m×0.25mm I.D., 0.25µm film thickness, Restek, USA) using helium as carrier gas at 40cm/sec in a constant linear velocity mode. Temperature program -50°C (1 min), 50°C →250 °C (25 °C/min), total run time - 15 min. The temperatures of injector, interface and ion source were 250°C. Detection was operated by selected ion monitoring (SIM) mode (EI mode), data was collected and analysed using the GC-MS solution version 2.71 (Kyoto, Japan).

For GC-MS analysis, the reaction mixture consisted of 45 mM potassium phosphate buffer (pH 7.5), 2.4 mM substrate (from 50-100 mM stock in DMSO), and protein (-0.5-1 mg/ ml). The total reaction mix volume was 1000 µl. The hydrolysis reaction products were extracted with ethyl acetate (3x 400 µl).

**GC-MS analysis results:** Reaction product extracted from reaction mixture after incubation of *N*⁴-benzoyl-2'-deoxycytidine with hydrolase (SEQ ID NO: 1) detected as benzamide MS(EI): *m*/*z* 51, 77, 105, 121. Reaction product extracted from reaction mixture after incubation of capecitabine with hydrolase (SEQ ID NO: 1) detected as pentyl-carbamate MS(EI): m/z 45, 55, 62, 70. Reaction product extracted from reaction mixture after incubation of 5-fluoro-*S*⁴-benzyl-thiouridine with hydrolase (SEQ ID NO: 1) detected as benzenmethanethiol, MS(EI): *mlz* 91, 124 and as spontaneous reaction product - benzyldisulfide MS(EI): *m*/*z* 91. Reaction product extracted from reaction mixture after incubation of 5-fluoro-*O*⁴-benzyloxyuridine with hydrolase (SEQ ID NO:1 detected as benzyl alcohol MS(EI): *m*/*z* 79, 108.

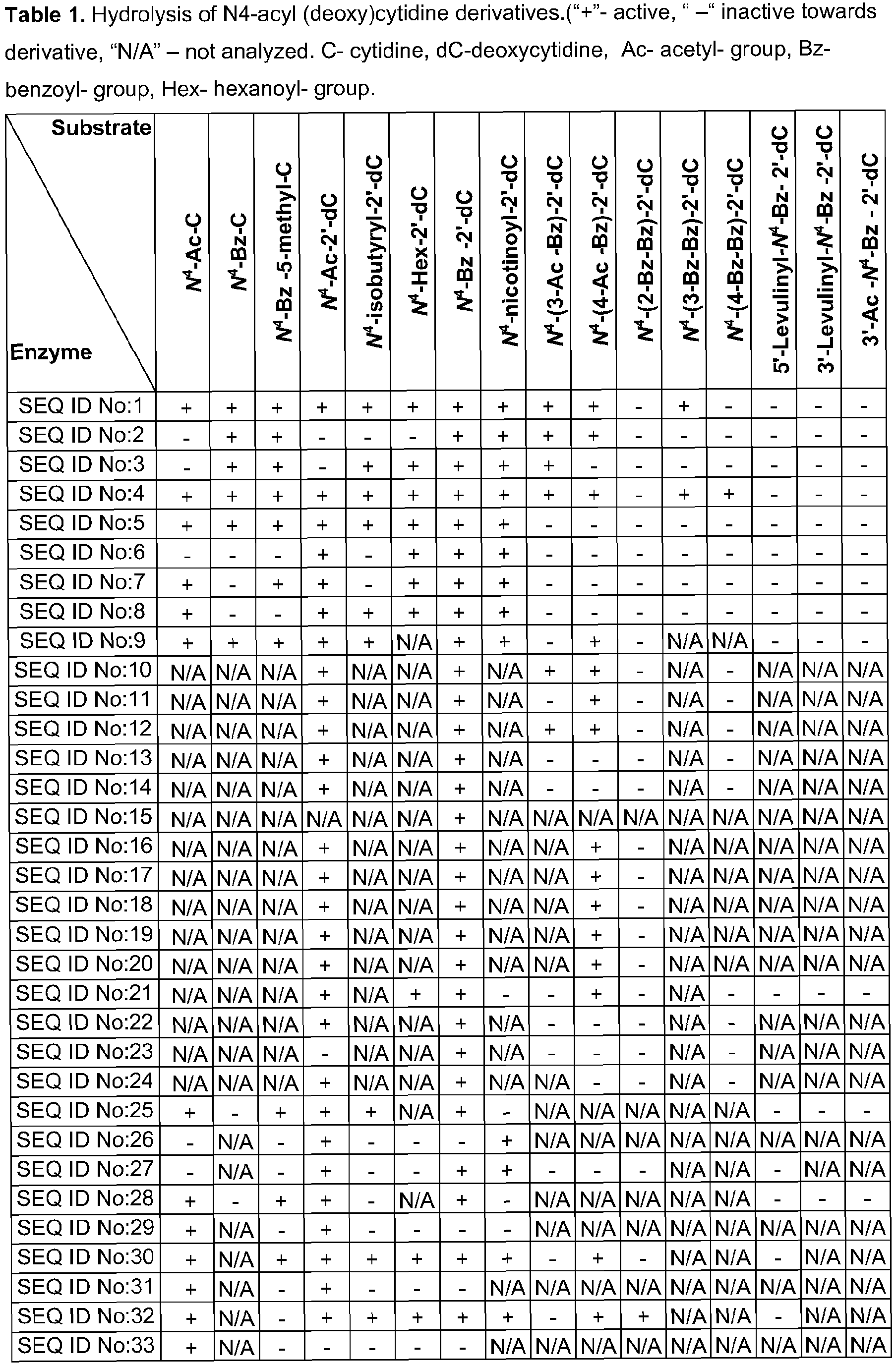

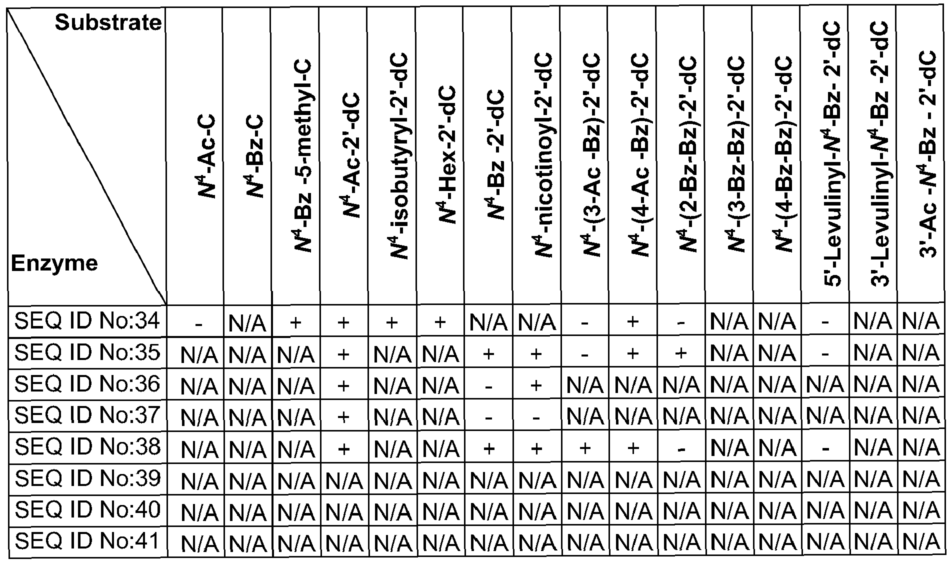

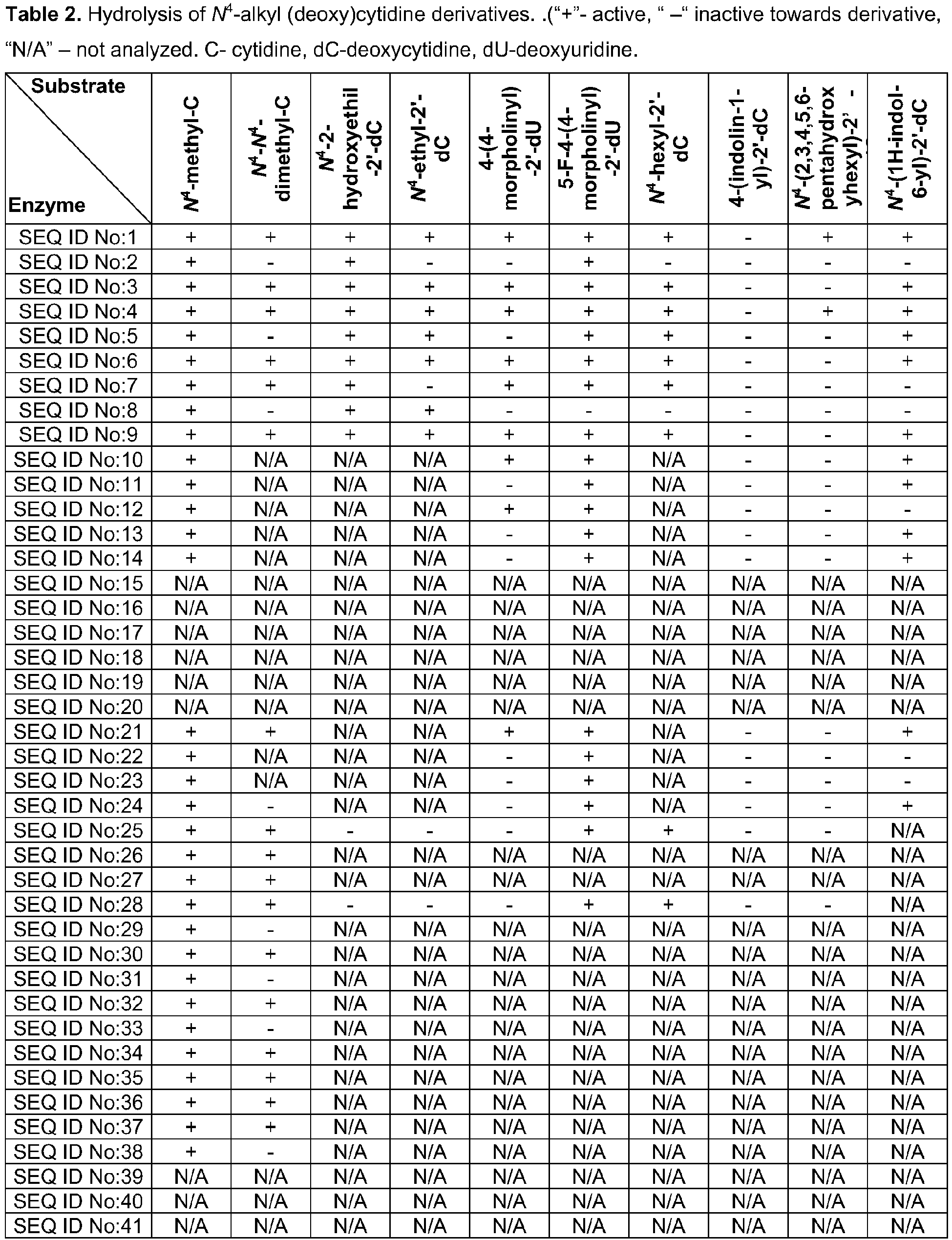

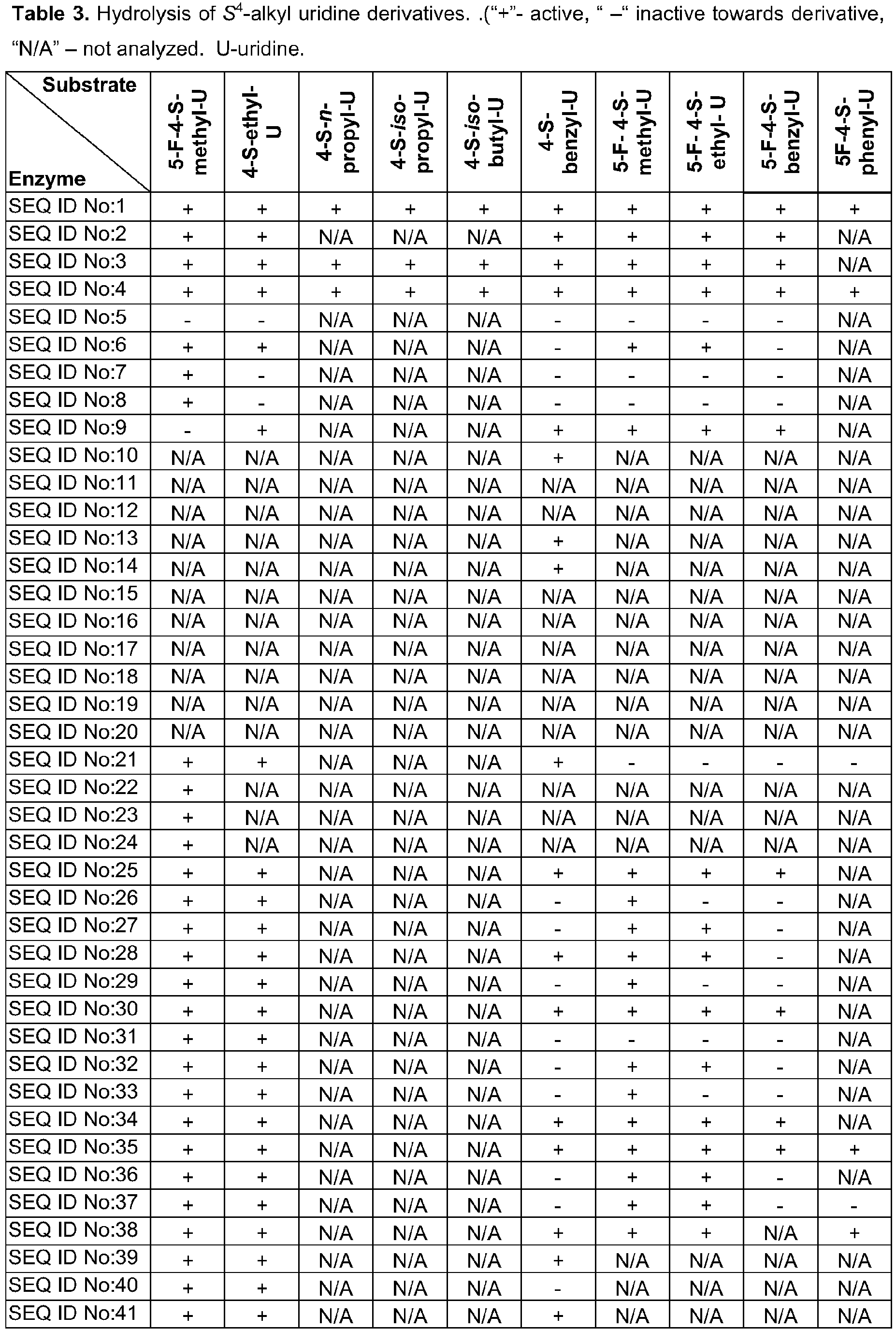

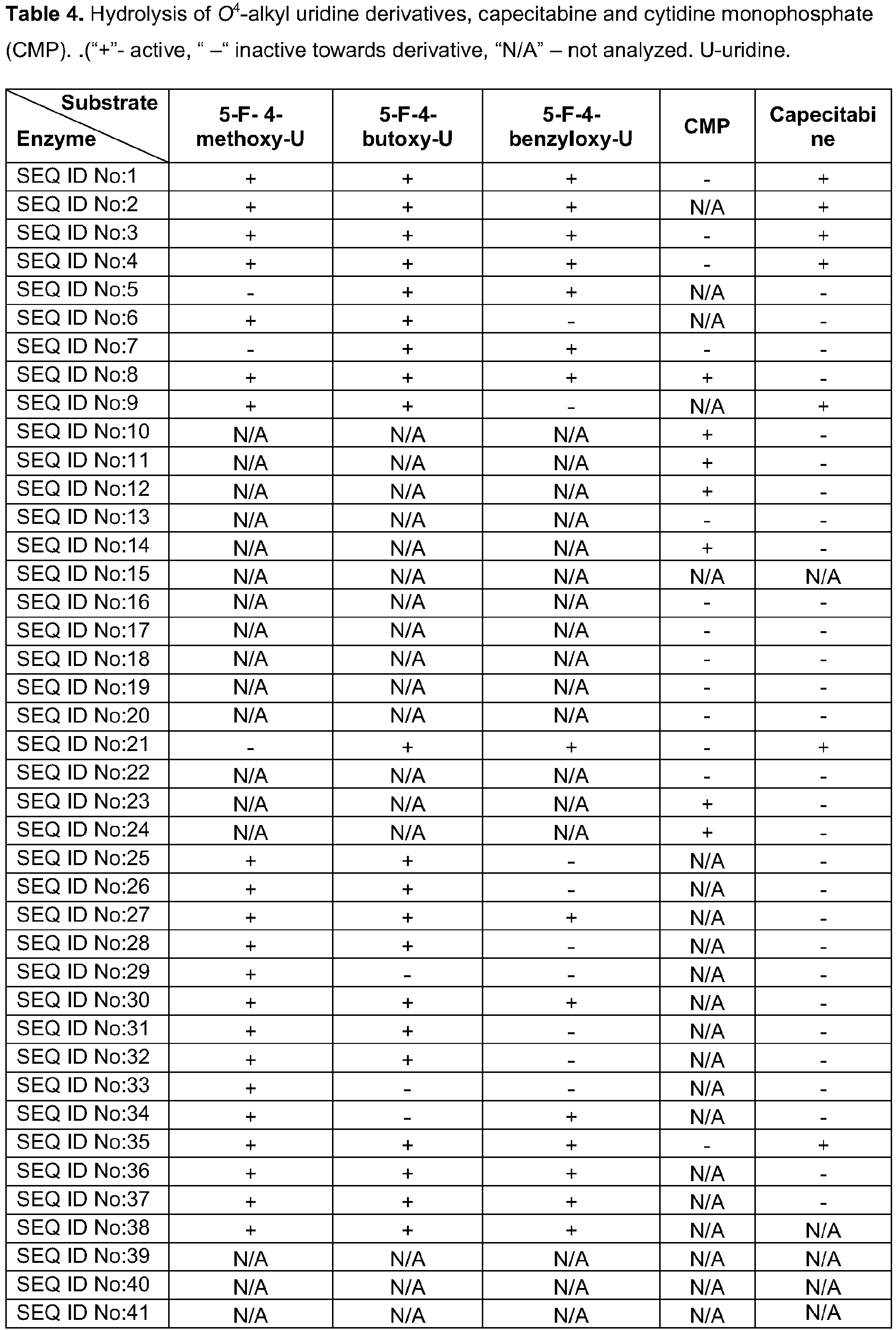

### Example 8. Spectrophotometric assay of hydrolytic activity of hydrolases

For the spectrophotometric assay, the reaction was started by adding an appropriate amount of protein to potassium phosphate buffer, pH 7.5 supplemented with 0.3, 0.15, 0.1, 0.075, 0.05, 0.0375, 0.025, 0.0125 mM *N*⁴-benzoyl-2'-deoxycytidine, or 1, 0.8, 0.5, 0.4, 0.25, 0.2, 0.1, 0.05 mM 2'-deoxycytidine. Then, decreases in absorbance at either 310 for *N*⁴-benzoyl-2'-deoxycytidine or 290 nm for 2'-deoxycytidine were recorded at 22 °C. Kinetic parameters (Table x) were calculated using Imfit-py 1.0.2 software fitting the experimental data to a simple Michaelis-Menten kinetics scheme.

**Table 5. Kinetic parameters of hydrolases. Substrate: BzdC - N⁴-benzoyl-2'-deoxycytidine; dC-2'-deoxycytidine.**

| Seq ID | **Substr ate** | **KM, (M)** | **Vmax,** | **kcat,( s⁻¹)** | **kcat/Km (M⁻¹/S⁻¹)** |
|---|---|---|---|---|---|
| Seq ID No: 1 | BzdC | (1.15 ± 0.16) × 10⁻⁴ | (1.00 ± 0.08) × 10⁻⁴ | (5.04 ± 0.4) ×10⁻¹ | (4.36 ± 3.6) ×10 ³ |
| | dC | (1.95 ± 0.36) × 10⁻⁴ | (2.67 ± 0.19) × 10⁻⁴ | (24.4 ± 1.71) × 10 ⁻¹ | (12.5 ± 1.2) ×10 ³ |
| Seq ID No: 3 | BzdC | (4.65 ± 0.68) ×10 ⁻⁵ | (6.55 ± 0.41) × 10⁻⁵ | (3.81 +/- 0.24) × 10 ⁻¹ | (81.9 +/- 6.04) × 10² |
| | dC | (1.82 ± 0.22) × 10⁻⁴ | (1.20 ± 0.05) × 10⁻⁴ | (2.09 +/- 0.09) ×10⁻¹ | (11.5 +/- 0.78) × 10² |
| Seq ID No: 10 | BzdC | (1.46 ± 0.01) × 10⁻⁴ | (5.49 ± 0.23) × 10⁻⁵ | (5.41 ± 0.23) × 10⁻² | (3.7 ± 0.09) × 10² |
| Seq ID No: 11 | BzdC | (2.59 ± 0.73) × 10⁻⁴ | (4.56 ± 0.093) × 10⁻⁵ | (3.88 ± 0.80) × 10⁻² | (1.5 ± 0.09) × 10² |
| Seq ID No: 12 | BzdC | (2.11 ± 0.38) × 10⁻⁴ | (1.19 ± 0.15) × 10⁻⁴ | (10.4 ± 1.3) ×10 ⁻ 2 | (4.92 ± 0.24) × 10² |
| Seq ID No:13 | BzdC | (1.56 ± 0.1) × | (2.17 ± 0.09) | (2.36 ± 0.01) × 10 ⁻¹ | (1.52 ± 0.08) × 10 ³ |
| | dC | (2.96 ± 0.88) × 10⁻⁴ | (4.35 ± 0.51) × 10⁻⁴ | (6.87 ± 0.80) × 10 ⁻¹ | (2.32 ± 0.32) × 10 ³ |
| Seq ID No: 15 | BzdC | (1.33 ± 0.17) × 10⁻⁴ | (3.17 ± 0.25) × 10⁻⁵ | (1.22 ± 0.09) × 10⁻² | (9.20 ± 0.42) × 10 ¹ |
| | dC | (1.67 ± 0.19) × 10⁻⁴ | (6.70 ± 0.24) × 10⁻⁵ | (2.58 ± 0.09) × 10⁻² | (15.5 ± 1.1) × 10 ¹ |

### Example 9. Activity detection in vivo in eukaryotic cells

**Cell lines.** All procedures were carried out under aseptic conditions meeting biological safety requirements. 293FT Cell Line (Gibco, Cat# R700-07) is derived from the 293F Cell Line and stably express the SV40 large T antigen from the pCMVSPORT6TAg.neo plasmid. HCT116 cells are human colon cancer cells derived from the colon of an adult male, colon cancer patient. 293FT cells were maintained in DMEM (Gibco) containing 10% fetal bovine serum (FBS, Gibco), 0.1 mM MEM Non-Essential Amino Acids (NEAA, Gibco), 6 mM L-glutamine (Gibco), 1 mM MEM Sodium Pyruvate (Gibco), 100 U/ml Penicillin and 0.1 mg/ml Streptomycin (Gibco), 0.5 mg/ml Geneticin. HCT116 cells were maintained in DMEM containing 10% FBS, 100 U/ml Penicillin and 0.1 mg/ml Streptomycin. Cells were grown at 37°C with 5% CO₂ in a water-saturated incubator. For passaging, cells were incubated with trypsin/EDTA (Corning) at 37°C to detach cells.

**Plasmids.** The DNA fragment, encoding His-tagged cytidine deaminase SEQ ID No: 4 flanked by the unique restriction sites BamHI and *Eco*105I, was cloned into vector pBABE-Puro (Cell Biolabs, Inc.) using *Bam*HI and *E*co105l restriction sites. Construct was sequence-verified by the Macrogen Europe (Netherlands).

**Retroviral Generation of Stable Cell Line.** Retroviral pBABE-Puro vector encoding the desired construct (1066.6 ng) were co-transfected with pCMV-gag-pol (355.6 ng) and pCMV-VSV-G (177.8 ng) (Cell Biolabs, Inc.) into 4 cm² size dish of -70% confluent 293FT cells. 100 µl Opti-MEM medium (Gibco) was mixed with 4 µl Lipofectamine 2000 reagent (Invitrogen) and incubated for 5 min at room temperature. Then, plasmids diluted in 100 µl Opti-MEM medium were added to the diluted Lipofectamine 2000. Following a gentle mix and incubation at room temperature for 20 min, the transfection mix was added dropwise to 293FT cells. 16 hr post-transfection, fresh medium was added to the cells. 24 hr later, the retroviral medium was collected and passed through 0.45 µm sterile syringe filters. Target HCT116 cells (~60% confluent) were transduced with the optimized titre of the retroviral medium diluted in fresh medium (typically 1:1-1:10) containing 8 µg/ml polybrene (Sigma-Aldrich) for 24 hr. The retroviral medium was then replaced with fresh medium, and 24 hr later, the medium was again replaced with fresh medium containing 2 µg/ml puromycin (Sigma-Aldrich) for selection of cells which had integrated the construct. A pool of transduced cells was utilized for subsequent experiments following complete death of non-transduced cells placed under selection in parallel.

**Preparation of solutions of prodrugs.** Solutions of prodrugs were prepared fresh for each experiment in DMSO diluted in the complete culture medium and added to HCT116 cells to a final concentration of 1-100 µM. The concentration of DMSO in the assay never exceeded 0.02% and had no influence on cell growth.

**MTT assay.** HCT116 cells were cultured on 96-well plates at a density of 4000 cells/well. The cells were incubated for 24 h to allow cells attach to the culture vessel before they were exposed to several concentrations of prodrugs for 24-48 h. After that, culture medium was carefully removed and 100 µl of the MTT solution (0.5 µg/ml MTT reagent (Merck) in PBS) was added to each well. The metabolically active cells reduced MTT to blue formazan crystals. After 1 h of incubation at 37°C, MTT-formazan crystals were dissolved in 120 µl DMSO, 100 µl of which was transferred to new 96-well plate suitable for optical measurements. The absorbance of the coloured formazan product was measured at 540 and 650 nm by Varioskan Flash Spectrophotometer (Thermo Fisher Scientific). The untreated cells and those treated with 5-FUR served as negative and positive control groups, respectively. Prior to performing statistical analysis, the obtained data was processed in three steps: first, the 650 nm measurements (background) were subtracted from each individual 540 nm measurement; the mean measurement value obtained from the samples treated with DMSO was subtracted from control group and each test cell sample group treated with either prodrug or 5-FUR; viability of the cells was evaluated by comparison of test samples against the negative control and expressed as percentage considering that viability of untreated cells was 100%. Each test group was tested in eight replicates (Figures 1-3).

**Statistical analysis.** All grouped MTT analysis data was presented as mean with 95% confidence interval. Comparisons between groups were made by the one-tailed Welch's t test for independent samples. The Shapiro-Wilk test was performed to evaluate deviation from normality. The significance level was chosen at α = 0.05 for all criteria used. Data were plotted and statistical analysis was performed using Rstudio version 1.3.1073.

### Example 10. Selection of lipases/esterases using uridine auxotrophic E. coli cells harbouring hydrolase gene

*E. coli* HMS174 Δ*pyr*FΔ*cdd* competent cells were transformed with the pLATE31 plasmids with gene of hydrolase of SEQ IDs NO: 1 - 9. The clones were additionally transformed with the compatible plasmids (p15 origin) harbouring metagenomic library and cultivated on the selective M9 medium agar plate containing 20 µg/ml kanamycin, 100 µg/ml ampicillin, and 20 µg/ml *N*⁴-2-hydroxyethyl-2'-deoxycytidine, in which 2-hydroxyethyl group was additionally acylated by the medium size fatty acid, as the sole source of uridine. The lipase/esterase converts acylated *N*⁴-2-hydroxyethyl-2'-deoxycytidine into *N*⁴-2-hydroxyethyl-2'-deoxycytidine, which is further converted by hydrolase of SEQ IDs NO: 1 - 9 to 2'-deoxyuridine, upon which the growth phenotype of uridine auxotrophic cells is restored. As a result, if the clone harbours a gene encoding the active lipase/esterase, colony growth is observed after 1-2 days of cultivation.

### Literature

1. Clark, J.L., Hollecker, L., Mason, J.C., Stuyver, L.J., Tharnish, P.M., Lostia, S., McBrayer, T.R., Schinazi, R.F., Watanabe, K.A., Otto, M.J., et al. (2005). Design, synthesis, and antiviral activity of 2'-deoxy-2'-fluoro-2'-C-methylcytidine, a potent inhibitor of hepatitis C virus replication. J. Med. Chem. 48, 5504-5508.
2. Cohen, R.M., and Wolfenden, R. (1971). Cytidine Deaminase from Escherichia coli PURIFICATION, PROPERTIES, AND INHIBITION BY THE POTENTIAL TRANSITION STATE ANALOG 3,4,5,6-TETRAHYDROURIDINE. J. Biol. Chem. 246, 7561-7565.
3. Gao, Y.; Zhang, P.; Wu, L.; Matsuura, T.; Meng, J. Synthesis of Uridine Derivatives Containing Amino Acid Residues. Synth. Commun. 2003, 33 (15), 2635-2641.
4. Goble, A.M., Fan, H., Sali, A., and Raushel, F.M. (2011). Discovery of a Cytokinin Deaminase. ACS Chem. Biol. 6, 1036-1040.
5. Gong, Y.; Chen, L.; Zhang, W.; Salter, R. Transglycosylation in the Modification and Isotope Labeling of Pyrimidine Nucleosides. Org. Lett. 2020, 22, 14, 5577-5581.
6. Jakubovska, J., Tauraite, D., Birštonas, L. & Meškys, R. Nucleic Acids Res. 46, 5911-5923 (2018)
7. Jansen RS, Rosing H, Schellens JH, Beijnen JH. Deoxyuridine analog nucleotides in deoxycytidine analog treatment: secondary active metabolites? Fundam Clin Pharmacol. 2011 Apr;25(2):172-85. doi: 10.1111/j.1472-8206.2010.00823.x. PMID: 20199587.
8. Kaleta, Z.; Makowski, B. T.; Soós, T.; Dembinski, R. Thionation Using Fluorous Lawesson's Reagent. Org. Lett. 2006, 8 (8), 1625-1628.
9. Kraszewski A., Delort A.M., Treoule R. (1986) cSynthesis of N4-mono- and dialkyl-2'-deoxycytidines and their insertion into an oligonucleotide, Tetrahedron Lett, 27, 861-864
10. Liu, J., Liu, J., Zhao, D., Ma, N., and Luan, Y. (2016). Highly enhanced leukemia therapy and oral bioavailability from a novel amphiphilic prodrug of cytarabine. RSC Adv. 6, 35991-35999.
11. Malekshah, O.M., Chen, X., Nomani, A. et al. Enzyme/Prodrug Systems for Cancer Gene Therapy. Curr Pharmacol Rep 2, 299-868 (2016). https://doi.org/10.1007/s40495-016-0073-y.
12. Milecki, J.; Nowak, J.; Skalski, B.; Franzen, S. 5-Fluoro-4-thiouridine phosphoramidite: New synthon for introducing photoaffinity label into oligodeoxynucleotides. Bioorg. Med. Chem. 2011, 19, 6098-6106.
13. Murakami, Y., Kimura, Y., Kawahara, A., Mitsuyasu, S., Miyake, H., Tohyama, K., Endo, Y., Yoshida, N., Imamura, Y., Watari, K., et al. (2019). The augmented expression of the cytidine deaminase gene by 5-azacytidine predicts therapeutic efficacy in myelodysplastic syndromes. Oncotarget 10, 2270-2281.
14. Nowak, I., and Robins, M.J. (2005). Hydrothermal Deamidation of 4-N-Acylcytosine Nucleoside Derivatives: Efficient Synthesis of Uracil Nucleoside Esters. Org. Lett. 7, 4903-4905.
15. Shelton, J., Lu, X., Hollenbaugh, J.A., Che, J.H., Amblard, F., and Schinazi, R.F. (2016). Metabolism, Biochemical Actions, and Chemical Synthesis of Anticancer Nucleosides, Nucleotides, and Base Analogs. Chem. Rev. 116, 14379-14455.
16. Thompson, J.D., Higgins, D.G., Gibson, T.J. CLUSTAL W: improving the sensitivity of progressive multiple sequence alignment through sequence weighting, position-specific gap penalties and weight matrix choice. Nucleic Acids Res. 1994 Nov 11;22(22):4673-80. doi: 10.1093/nar/22.22.4673.
17. Urbeliené, N. et al. A Rapid Method for the Selection of Amidohydrolases from Metagenomic Libraries by Applying Synthetic Nucleosides and a Uridine Auxotrophic Host. Catalysts 10, 445 (2020).
18. Urbelienė, N.; Kutanovas, S.; Meškienė, R.; Gasparavičiu̅tė, R.; Tauraite, D.; Koplu̅naitė, M.; Meškys, R. Application of the uridine auxotrophic host and synthetic nucleosides for a rapid selection of hydrolases from metagenomic libraries. Microb. Biotechnol. 2019, 12, 148-160.
19. Walko, C.M., and Lindley, C. (2005). Capecitabine: A review. Clin. Ther. 27, 23-44.
20. Wenska, G.; Taras-Goslinska, K.; Lamparska-Kupsik, K.; Skalski, B.; Gdaniec, M.; Gdaniec, Z. Photochemical transformations of 5-halogeno-4-thiouridines. J. Chem. Soc., Perkin Trans. 1, 2002, 53-57.
21. US010035983;
22. WO2009148048;
23. US8349318;
24. US20080206221;
25. LT6626;
26. WO2000047726;
27. WO2005049846;
28. WO2006103462;
29. WO2015075475;
30. WO9512678;
31. WO9945126.

### List of sequences

| | |
|---|---|
| SEQ ID No:1 | MNKEDLLKKAFEAMENAYAPYSNYHVGACALMKDGTTFLGANIENASYGATN CGERSAIFAAYSNGYRADDIEALAIVTDGDRVGAPCGICRQVLSELLNDNTPIY LSNGKETLEKTIDELLPMRFTKEDLL |
| SEQ ID No:2 | MRHDLVASAMAAREHARAAYSGFKVGAAIRTQDGAIFSGCNIENATLGLTVC AERVALWKALSEGADSFTEIVIATDSDNGGPCGACRQLLWEYCGDIAVRFHSI AGRDQMFRLSELLPHPFDANSLG |
| SEQ ID No:3 | MKETLCQEARKARENAYCPYSRFMVGAAVLGEDGRIYTGCNIENTSYGMTV CAERTAIFKMVSEGCRKFKALAVAGSTEADGAPCMACRQVMTEFCFDMDVP VYVTCPSGKIYTHTMKEICPYPFMAFDPSEDYGK |
| SEQ ID No:4 | MRDKLIAEAWEGQKNSYSPYSKFPVGAAVMGADGVIYRGCNIENVSYGLTVC GERNAIFNMVMHGCQTYTALAVVCNGNADAAPCGACRQVMGEFCESLDTP VYICGGEGNLLETTVGELMPYPFLKFDPAK |
| SEQ ID No:5 | MYENLVSEAFEAMKSAYAPYSNYHVGACVLCRDGRTFRGVNIENASYGATN CAERSAVFGAYSYGYRKADIAALAIVSDGARIGTPCGICRQVLSELLESDTPIIL SNGKETMETTMAELLPMSFGMEDLA |
| SEQ ID No:6 | MTREELIEEAFKAMENSYAPYSNFHVGAAVLTKDGKVFWGANIENASFGATN CGERSAIFAAYSNGYRKETIEALAIVSDGDRIAAPCGICRQVLSELITGDTPIYL SNRKDEMDTCINELLPYQFTTEDVCR |
| SEQ ID No:7 | MNRDDLIKKAREAKEFAYSPYSNFKVGAAILTENGEVFTGCNIENISYGATNC AERTAIFKAISDGNKSFKEIAISSSIAPEKPIFPCGICRQVLAEFNKDMLIHVDGD DKTYKLSELLPAPFDKNQMSNF |
| SEQ ID No:8 | MKDQLIQKALEARKQAYVPYSNFQVGAAVLGSDGTVYHGCNVENASYGLCN CAERTAIFKMVSEGCRKIDSIAVAADTEGPVSPCGACRQVISEFAHQDTKIYLT NLHGNTEEWTMEQLLPGAFRPADLGK |
| SEQ ID No:9 | MNRQELITEALKARDMAYAPYSKFQVGAALLTKDGKVYRGCNIENAAYSMCN CAERTALFKAVSEGETEFQMLAVAADTPGPVSPCGACRQVISELCTKDVIVVL TNLQGQIKEMTVEELLPGAFSSEDLHDERKL |
| SEQ ID No:10 | MNKEDLLKKAFEAMENAYAPYSNYHVGACALMKDGTTFLGANIENASYGATN CGERSAIFAAYSNGYRADDIEALAIVTDGDRVGAPCGICRQVLSELLNDNTPIY LSNGKETLEKTIDELLPMRFLL |
| SEQ ID No:11 | MNKEDLLKKAFEAMENAYAPYSNYHVGACALMKDGTTFLGANIENASYGATN CGERSAIFAAYSNGYRADDIEALAIVTDGDRVGAPCGICRQVLSELLNDNTPIY LSNGKETLEKTIDELLPMRATKEDLL |
| SEQ ID No:12 | MNKEDLLKKAFEAMENAYAPYSNYHVGACALMKDGTTFLGANIENASYGATN CGERSAIFAAYSNGYRADDIEALAIVTDGDRVGAPCGICRQVLSELLNDNTPIY LSNGKETLEKTIDELLPMRWTKEDLL |
| SEQ ID No:13 | MNKEDLLKKAFEAMENAYAPYSNYHVGACALMKDGTTFLGANIENASYGATN CGERSAIFAAYSNGYRADDIEALAIVTDGDRVIAPCGICRQVLSELLNDNTPIYL SNGKETLEKTIDELLPMRFTKEDLL |
| SEQ ID No:14 | MNKEDLLKKAFEAMENAYAPYSNYHVGACALMKDGTTFLGANIENASYGAG NCGERSAIFAAYSNGYRADDIEALAIVTDGDRVGAPCGICRQVLSELLNDNTPI YLSNGKETLEKTIDELLPMRFTKEDLL |
| SEQ ID No:15 | MNKEDLLKKAFEAMENAYAPYSNYHVGACALMKDGTTFLGANIENASYGATN CGELSAIFAAYSNGYRADDIEALAIVTDGDRVGAPCGICRQVLSELLNDNTPIY LSNGKETLEKTIDELLPMRFTKEDLL |
| SEQ ID No:16 | MNKEDLLKKAFEAMENAYAPYSNYHVGACALMKDGTTFLGANIENASYGATN CGERSAIFAAYSNGYRADDIEALAIVTDGDQQSAPCGICRQVLSELLNDNTPIY LSNGKETLEKTIDELLPMRFTKEDLL |
| SEQ ID No:17 | MNKEDLLKKAFEAMENAYAPYSNYHVGACALMKDGTTFLGANIENASYGATN CGERSAIFAAYSNGYRADDIEALAIVTDGDSMLAPCGICRQVLSELLNDNTPIY LSNGKETLEKTIDELLPMRFTKEDLL |
| SEQ ID No:18 | MNKEDLLKKAFEAMENAYAPYSNYHVGACALMKDGTTFLGANIENASYGATN CGERSAIFAAYSNGYRADDIEALAIVTDGDHSIAPCGICRQVLSELLNDNTPIYL SNGKETLEKTIDELLPMRFTKEDLL |
| SEQ ID No:19 | MNKEDLLKKAFEAMENAYAPYSNYHVGACALMKDGTTFLGANIENASYGATN CGERSAIFAAYSNGYRADDIEALAIVTDGDRVGAPCGICRQVLSELLNDNTPIY LSNGKETLEKTIDELLPMRFHSSGLL |
| SEQ ID No:20 | MNKEDLLKKAFEAMENAYAPYSNYHVGACALMKDGTTFLGANIENASYGATN CGERSAIFAAYSNGYRADDIEALAIVTDGDRVGAPCGICRQVLSELLNDNTPIY LSNGKETLEKTIDELLPMRFCLYRLL |
| SEQ ID No:21 | MRDKLIAEAWEGQKNSYSPYSKFPVGAAVMGADGVIYRGCNIENVSYGLTVC GERNAIFNMVMHGCQTYTALAVVCNGNADIAPCGACRQVMGEFCESLDTPV YICGGEGNLLETTVGELMPYPFLKFDPAKE |
| SEQ ID No:22 | MKDQLIQKALEARKQAYVPYSNFQVGAAVLGSDGTVYHGCNVENASYGLTN CAERTAIFKMVSEGCRKIDSIAVAADTEGPVSPCGACRQVISEFAHQDTKIYLT NLHGNTEEWTMEQLLPGAFRPADLGK |
| SEQ ID No:23 | MKDQLIQKALEARKQAYVPYSNFQVGAAVLGSDGTVYHGCNVENASYGLCN CAERTAIFKMVSEGCRKIDSIAVAADTEGPLSPCGACRQVISEFAHQDTKIYLT NLHGNTEEWTMEQLLPGAFRPADLGK |
| SEQ ID No:24 | MNRDDLIKKAREAKEFAYSPYSNFKVGAAILTENGEVFTGCNIENISYGATNC AERTAIFKAISDGNKSFKEIAISSSIAPEKPAFPCGICRQVLAEFNKDMLIHVDG DDKTYKLSELLPAPFDKNQMSNF |
| SEQ ID No:25 | MILLSEAKKKELIELAKEASRKAYAPYSKFKVGVALLAQNGKIYQGCNVENAS YSMTICAERNAVFQAVADGNKDFAAIFIYVDSDKSFPPCGACRQVLAEFAGD MLIFIGNRNTVSETTLIEILPERFTLTDE |
| SEQ ID No:26 | MVTKNISITWTEVNSIEELDKSDRELVLAAREAAQNAHAPYSGFRVGAAVRLA SGIIVRGANIENAAFPSGICAERSALSNSASNYPAIKPVALAVAAFTEDGMTQE PVPPCGNCRQVIAEEEFRNNNNIRIILSGENKIQIIEKGGDLLPLQFSKNYLKIKP L |
| SEQ ID No:27 | MSADERTKNGTPPEQPDDRTLYETAVKMRERSYAPYSRFRVGAALLAKDGM VFTGCNIENASYGACNCAERTAFFKAVSEGVRQFAAIAIAGGPDGAEELDFCP PCGICRQVMEEFCSADFRIILGNTSGELKCLTLAELLPYGFGPGDLDK |
| SEQ ID No:28 | MKKISITTTISETNSLNDLGIEDQEIISLALSAAGASWSPYSRFAVGAALRLANG VIVTGTNVENAAFPSGICAEHTALSTAASSYPEIAPVAMAITALNKGKQVVTPV APCGKCRQVIAETENRYKRDIKLFLVGQNKVTIIERGCDLLPLVFSQRDLSSSD E |
| SEQ ID No:29 | MSKVSDQDLIRLAMEAREYSYSPYSHFMVGAALLAKNGKVYQGCNIENAGYT PSNCAERTAFFKAVSEGVLEFETIAIVAGPKDGELIRTAPCGVCRQVMMEFCE YKTFRVLLAKSPEDYEEWTLEKLLPFGFGPKDLA |
| SEQ ID No:30 | MTDRRITIDFTEYSSPDEMEPQDRELVAAALRARTGSYSPYSRFRVGAALRL VDGTVVLGANQENVAYPSGLCAERTAMFAAGANHPGVAFDTLAIVGANGDE VCEMPAAPCGACRQVMAEYQRLHGRPLKVILIGTHAIYKFAKVEDLLPLIFDSL KVE |
| SEQ ID No:31 | MRTKEDFRPLADAAIDASRQSYSPYSQFKVGAAILTADRKVYQGTNVENASY GGTICAERTALVKLVSEERHTMEARAPRTYIEAIAVASNLRGEAVCPCGMCLQ FMQEFMSGHEPIILVNRDTRVIEQVLTLQELLPHAFRLLD |
| SEQ ID No:32 | MTDWTYLIDKAFEARENAYAPYSNFQVGACVETKDGCFIPGANVENAAYGST ICGERNAIFGAYARGYRKEDIKALAIVTGAERISTPCGSCRQVLSELLEPDTPIV LCNRKDTVVTNIAELLPGAFGAEDLAK |
| SEQ ID No:33 | MDLERSRTLFQTIGGEPINNDLVVTMIAQAREARAFSYAPYSNYNVGCAALLE RDGKRSIFRGTNIENAAYWPTMCAERSAIFSAASLGFRKLLAVAVVGDRDDT SLVTPCGGCRQVMNEFGNALTLIIMAKEDDTVELRTLGQLLPEAFGPSNLGM NPQNYMAKK |
| SEQ ID No:34 | MTEKKITTKVRVYSYNELTEEQKKLVDLAREATSHSYSPYSNFKVGAALMLDN GEVFIGANQENAAFAGICGERAALYAAGAKHPGVPVKSVAITSFTRDAFVEEP TAPCGVCRQAFLEFEKNGHPIELILAGKEKIYILDSFKDTMPLSFTEF |
| SEQ ID No:35 | MNQLTQQEMDLLLDKAQEARDHSYAPYSKYNVGAALMTADGQIYQGCNIEN AGFTPTVCAERTAFFKAVYDGHRAFRAIAVIATGEELGFPCGVCRQVMAEFC DRDFIIVTANRDRTKVDVSDFETLLPHSFGPKDLGV |
| SEQ ID No:36 | MTNVTPEDAKFLIDLANQARERAYAPYSNYHVGAALRTKSGRIFTGVNVENA AYPTTMCAERVAIYKAVSEGELEFDVIAVVTSNGGSPCGGCRQVMAEFGLET VVLIGNGAGEILEETTVNGLLPGAFTPAHLKS |
| SEQ ID No:37 | MTETDKKLIAEAKRVREFAYCPYSKFAVGAAVLGASGEIYGGCNIENASFSVT NCAERTAIFKAVSAGEDDILAIAVVAEGVNPVPPCGACRQVIAEFNIPHILMAN LADEVKEMTLEELLPGAFSAEDING |
| SEQ ID No:38 | MDAGLLMQEAIKARTRAYIPYSKFGVGAALLDSRGQVHLGCNVENAAYGPTN CAERTALFRAIADGHEAGSFQAIAIVADTDGPCTPCGVCRQVLVELCKPDMK VIMGNMKGDIKETTVSELLPGAFGPWDLNKQ |
| SEQ ID No:39 | MDAGLLMQEAIKARMRAYIPYSTFGVGAALLDSHGEVHLGCNVENAAYGPTN CAERTALFRAIADGHEAGSFQAIAIVADTEGPCTPCGVCRQVLVELCKPDMKV IMGNMKGDIKETTVIELLPGAFGPWDLNKQ |
| SEQ ID No:40 | MNKQELISEAVKARDFAYVPYSKFKVGAALLSHDGKVYGGCNIENAAYGMCN CAERTALFKAYSEGITSFQMLAVVADTDRPVSPCGACRQVISELCAPDMPVIL TNLKGHIYETTVNELLPGAFSPEDLND |
| SEQ ID No:41 | MIAAASRAREFSYAPYSNFTVGAALITKSGKVFTGCNVESVSYSPTTCAERVA ILKAVSEGERSFEMIVVIGGPRVGESKAKGYSGPCGVCRQMIYEFGKDIQIIIA NSLDEYYVHDISELFPLGFGPDHLLG |

## Claims

1. An isolated hydrolase encoding DNA with an amino acid sequence from the group SEQ ID NO: 1-41 hydrolyses a pyrimidine derivative to a uridine derivative *in vitro* without organic solvents under ambient pressures and temperatures.

2. A method for using hydrolases according to claim 1, wherein the method comprises the steps of:
a) the nucleic acid selection from the group consisting of SEQ ID NO: 1-41;
b) construction of a DNA vector harbouring the selected nucleic acid;
c) host cell transformation with the DNA vector from step (b);
d) hydrolysis of a pyrimidine derivative to a uridine derivative.

3. The method according to claim 2, wherein the uridine derivatives prepared from cytidine derivatives, have the following general formula (1): 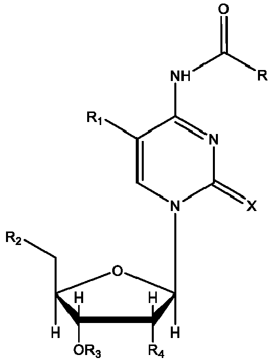
wherein R is C1-C10 alkyl, C1-C10 substituted alkyl, -(CH₂)n-Ph, or substituted -(CH₂)n-Ph;
wherein in said -(CH₂)n-Ph, n = 0, 1, 2, 3 to 10, Ph is phenyl; a carbon chain of said substituted alkyl is independently substituted by one or two or three hydroxyl group or carboxyl group; in said substituted -(CH₂)n-Ph, n = 0, 1, 2, 3 to 10, and a carbon chain or a phenyl ring of which is independently substituted by one or two or hydroxyl group or carboxyl group;
wherein X is S, O;
wherein R₁ is C1-C10 alkyl, C1-C10 substituted alkyl or F;
wherein R₂ is OH, -PO₄²⁻, C1-C10 alkyl, C1-C10 substituted alkyl;
wherein R₃: H;
wherein R₄: H, OH, C1-C10 alkyl, C1-C10 substituted alkyl or F;
substituents R₂, R₃, and R₄ can be identical or different.

4. The method according to claim 2, wherein the uridine derivatives prepared from pyrimidine derivatives, have the following general formula (**4**): 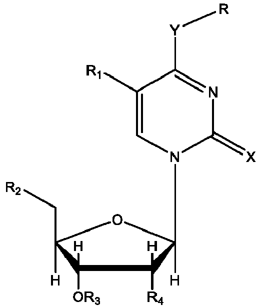
wherein Y is N(H), S or O;
wherein R is C1-C10 alkyl, C1-C10 substituted alkyl, -(CH₂)n-Ph, or substituted -(CH₂)n-Ph; wherein in said -(CH₂)n-Ph, n = 0, 1, 2, 3 to 10, Ph is phenyl; a carbon chain of said substituted alkyl is independently substituted by one or two or three hydroxyl group or carboxyl group; in said substituted -(CH₂)n-Ph, n = 0, 1, 2, 3 to 10, and a carbon chain or a phenyl ring of which is independently substituted by one or two or hydroxyl group or carboxyl group;
wherein X is S, O;
wherein R₁ is C1-C10 alkyl, C1-C10 substituted alkyl or F;
wherein R₂ is -OH, -PO₄²⁻, C1-C10 alkyl, C1-C10 substituted alkyl;
wherein R₃: H;
wherein R₄: H, OH, C1-C10 alkyl, C1-C10 substituted alkyl or F
substituents R₂, R₃, and R₄ can be identical or different.

5. The method according to claim 2, wherein the pseudoisouridine derivatives prepared from pseodoisocytidine derivatives, have the following general formula (**6**): 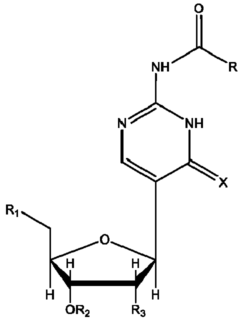
wherein R is C1-C10 alkyl, C1-C10 substituted alkyl, -(CH₂)n-Ph, or substituted -(CH₂)n-Ph;
wherein in said -(CH₂)n-Ph, n = 0, 1, 2, 3 to 10, Ph is phenyl; a carbon chain of said substituted alkyl is independently substituted by one or two or three hydroxyl group or carboxyl group; in said substituted -(CH₂)n-Ph, n = 0, 1, 2, 3 to 10, and a carbon chain or a phenyl ring of which is independently substituted by one or two or hydroxyl group or carboxyl group;
wherein X is S, O;
wherein R₁ is -OH, -PO₄²⁻, C1-C10 alkyl, C1-C10 substituted alkyl;
wherein R₂: H;
wherein R₃: H, OH, C1-C10 alkyl, C1-C10 substituted alkyl or F
substituents R₁, R₂, and R₃ can be identical or different.

6. The method according to claim 2, wherein the pseudoisouridine derivatives prepared from pseudoisocytidine derivatives, have the following general formula (**8**): 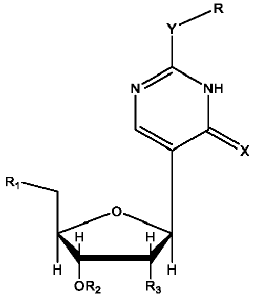
wherein R is C1-C10 alkyl, C1-C10 substituted alkyl, -(CH₂)n-Ph, or substituted -(CH₂)n-Ph;
wherein in said -(CH₂)n-Ph, n = 0, 1, 2, 3 to 10, Ph is phenyl; a carbon chain of said substituted alkyl is independently substituted by one or two or three hydroxyl group or carboxyl group; in said substituted -(CH₂)n-Ph, n = 0, 1, 2, 3 to 10, and a carbon chain or a phenyl ring of which is independently substituted by one or two or hydroxyl group or carboxyl group;
wherein Y is N, S or O;
wherein X is O, S;
wherein R₁ is -OH, -PO₄²⁻, C1-C10 alkyl, C1-C10 substituted alkyl;
wherein R₂: H;
wherein R₃: H, OH, C1-C10 alkyl, C1-C10 substituted alkyl or F;
substituents R₁, R₂, and R₃ can be identical or different.

7. The method according to claim 2, wherein the uridine derivatives prepared from cytidine derivatives, have the following general formula (9): 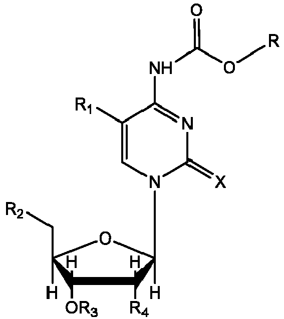
wherein R is C1-C10 alkyl, C1-C10 substituted alkyl, -(CH₂)n-Ph, or substituted -(CH₂)n-Ph;
wherein in said -(CH₂)n-Ph, n = 0, 1, 2, 3 to 10, Ph is phenyl; a carbon chain of said substituted alkyl is independently substituted by one or two or three hydroxyl group or carboxyl group; in said substituted -(CH₂)n-Ph, n = 0, 1, 2, 3 to 10, and a carbon chain or a phenyl ring of which is independently substituted by one or two or hydroxyl group or carboxyl group;
wherein X is O, S;
wherein R₁ is C1-C10 alkyl, C1-C10 substituted alkyl or F;
wherein R₂ is -OH, -PO₄⁻, C1-C10 alkyl, C1-C10 substituted alkyl;
wherein R₃: H;
wherein R₄: H, OH, C1-C10 alkyl, C1-C10 substituted alkyl or F;
substituents R₂, R₃, and R₄ can be identical or different.

8. The method according to claims 2-7, wherein the cytidine derivatives are hydrolysed *in vitro.*

9. The method according to claims 2-7, wherein the cytidine derivatives are hydrolysed in prokaryotic cells *in vivo.*

10. The method according to claims 2-7, wherein the cytidine derivatives are hydrolysed in eukaryotic cells *in vitro.*

11. The method according to claims 2-10, wherein the hydrolases are used for genome editing with CRISPR-Cas9 system.

12. A composition comprising a mixture of the hydrolases according to claim 1 and cytidine derivatives according to claims 2-7 for inhibition of the proliferation of a pathological cell, wherein the hydrolases are overexpressed in the cell, comprising contacting the cell with an effective concentration of the cytidine derivatives of 1-100 µM.

13. A method for the selection of enzymes using hydrolases according to claim 1, wherein the method comprises the steps of:
a) nucleic acid selection;
b) construction of a library of DNA vectors harbouring the selected nucleic acids;
c) transformation of the host cells harbouring a hydrolase from the group consisting of SEQ ID NO: 1-41 with the DNA vectors harbouring the selected nucleic acids;
d) enzyme selection from genetic libraries using the uridine, cytidine, cytosine and uracil depleted medium containing a pyrimidine compound that is converted by the enzyme into a substrate of the hydrolase from the group consisting of SEQ ID NO: 1-41.
